# EUROPEAN PATENT SPECIFICATION

(11) **EP 1 853 714 B1**
(45) Date of publication and mention of the grant of the patent: **26.11.2008**
(21) Application number: 06703761.4
(22) Date of filing: 18.01.2006
(51) Int. Cl.: C12P 13/04

(54) **METHOD FOR PRODUCING L-AMINO ACIDS USING BACTERIUM OF THE ENTEROBACTERIACEAE FAMILY**
VERFAHREN ZUR HERSTELLUNG VON L-AMINOSÄUREN UNTER VERWENDUNG EINES BAKTERIUMS DER FAMILIE ENTEROBACTERIACEAE
PROCEDE DE PRODUCTION DE L-AMINOACIDES AU MOYEN DE BACTERIES DE LA FAMILLE ENTEROBACTERIACEAE

(30) Priority: 19.01.2005 RU 2005101111
(43) Date of publication of application: 14.11.2007
(73) Proprietor: Ajinomoto Co., Inc., Tokyo 104-8315 (JP)
(72) Inventor: PTITSYN, Leonid Romanovich, Moscow 115404 (RU); ALTMAN, Irina, Borisovna, Moscow 119435 (RU); KOTLIAROVA, Veronika Aleksandrovna, Moscow 117574 (RU); KOZLOV, Yury Ivanovich, Moscow 117574 (RU)
(74) Representative: HOFFMANN EITLE
(86) International application number: PCT/JP2006/301090
(87) International publication number: WO 2006/078051

(56) References cited:
- WO-A-03/004662
- PLUMBRIDGE J A: "REPRESSION AND INDUCTION OF THE NAG REGULON OF ESCHERICHIA-COLI K-12 THE ROLES OF NAG-C AND NAG-A IN MAINTENANCE OF THE UNINDUCED STATE" MOLECULAR MICROBIOLOGY, vol. 5, no. 8, 1991, pages 2053-2062, XP008064325 ISSN: 0950-382X
- ROGERS M J ET AL: "NUCLEOTIDE SEQUENCES OF THE ESCHERICHIA COLI NAGE AND NAGB GENES: THE STRUCTURAL GENES FOR THE N-ACETYLGLUCOSAMINE TRANSPORT PROTEIN OF THE BACTERIAL PHOSPHOENOLPYRUVATE: SUGAR PHOSPHOTRANSFERASE SYSTEM AND FOR GLUCOSAMINE-6-PHOSPHATE DEAMINASE" GENE, ELSEVIER, AMSTERDAM, NL, vol. 62, January 1988 (1988-01), pages 197-207, XP002004349 ISSN: 0378-1119

### SEQUENCE LISTING

<110> Ajinomoto Co., Inc.
<120> METHOD FOR PRODUCING L-AMINO ACIDS USING BACTERIUM OF THE ENTEROBACTERIACEAE FAMILY
<130> C360-C5322
<150> RU2005101111
   <151> 2005-01-19
<160> 30
<170> PatentIn version 3.3
<210> 1
   <211> 1947
   <212> DNA
   <213> Escherichia coli
<220>
   <221> CDS
   <222> (1)..(1947)
<400> 1 <210> 2
   <211> 648
   <212> PRT
   <213> Escherichia coli
<400> 2
<210> 3
   <211> 70
   <212> DNA
   <213> Artificial
<220>
   <223> primer
<400> 3
   gatgaaagcg ttatccaaac tgaaagcgga agaggccgac gcactttgcg ccgaataaat 60
   acctgtgacg 70
<210> 4
   <211> 69
   <212> DNA
   <213> Artificial
<220>
   <223> primer
<400> 4
   ttaatcccag ctcagaataa ctttcccgga ctttacgccc cgccctgcca ctcatcgcag 60
   tactgttgt 69
<210> 5
   <211> 18
   <212> DNA
   <213> Artificial
<220>
   <223> primer
<400> 5
   cggtcatgct tggtgatg 18
<210> 6
   <211> 21
   <212> DNA
   <213> Artificial
<220>
   <223> primer
<400> 6
   ttaatcccag ctcagaataa c 21
<210> 7
   <211> 30
   <212> DNA
   <213> Artificial
<220>
   <223> primer
<400> 7
   ccaagatctg gagcttatcg actgcacggt 30
<210> 8
   <211> 71
   <212> DNA
   <213> Artificial
<220>
   <223> primer
<400> 8
   acgccctacc gagtcgctgg aaaaaaccta aaatattcat ggtctgtttc ctgtgtgaaa 60
   ttttatccgc t 71
<210> 9
   <211> 69
   <212> DNA
   <213> Artificial
<220>
   <223> primer
<400> 9
   tcacacactc tgtagcagat gatctaacaa tctgattaca gtcagaaaaa ctcatcgagc 60
   atcaaatga   69
<210> 10
   <211> 30
   <212> DNA
   <213> Artificial
<220>
   <223> primer
<400> 10
   ttcagatctg ttgtgtctca aaatctccga 30
<210> 11
   <211> 25
   <212> DNA
   <213> Artificial
<220>
   <223> primer
<400> 11
   ctgacctggc ctgctttatg cattt 25
<210> 12
   <211> 25
   <212> DNA
   <213> Artificial
<220>
   <223> primer
<400> 12
   aatctggctg acgcaatcgc agcaa 25
<210> 13
   <211> 69
   <212> DNA
   <213> Artificial
<220>
   <223> primer
<400> 13
   tccacgagat gcggcccaat ttacgtctta ggagaagatc aactaagcac ttgtctcctg 60
   tttactccc 69
<210> 14
   <211> 69
   <212> DNA
   <213> Artificial
<220>
   <223> primer
<400> 14
   aatggcgccg atgggcgcca tttttcactg cggcaagaat tgctgctttt aagacccact 60
   ttcacattt 69
<210> 15
   <211> 24
   <212> DNA
   <213> Artificial
<220>
   <223> primer
<400> 15
   agcaggctct tgctcaaccg acaa 24
<210> 16
   <211> 24
   <212> DNA
   <213> Artificial
<220>
   <223> primer
<400> 16
   ggaagtgatg cgctacaccc agca 24
<210> 17
   <211> 33
   <212> DNA
   <213> Artificial
<220>
   <223> primer
<400> 17
   cacaaagctt ggttctcgta ggaggaataa gat 33
<210> 18
   <211> 32
   <212> DNA
   <213> Artificial
<220>
   <223> primer
<400> 18
   tgtgtctaga cctcactcat cgtggattcc tc 32
<210> 19
   <211> 180
   <212> DNA
   <213> Artificial
<220>
   <223> hybrid promoter
<400> 19
   ctagatctct cacctaccaa acaatgcccc cctgcaaaaa ataaattcat aaaaaacata     60
   cagataacca tctgcggtga taaattatct ctggcggtgt tgacaattaa tcatcggctc    120
   gtataatgtg tggaattgtg agcgggttct cgtaggggga ataagatgaa tattttaggt    180
<210> 20
   <211> 32
   <212> DNA
   <213> Artificial
<220>
<223> primer
<400> 20
   gtaagatctc tcatgtttga cagcttatca tc 32
<210> 21
   <211> 52
   <212> DNA
   <213> Artificial
<220>
   <223> primer
<400> 21
   cctaaaatat tcatcttatt ccccctacga gaacccctaa gctttctaga cg 52
<210> 22
   <211> 52
   <212> DNA
   <213> Artificial
<220>
   <223> primer
<400> 22
   acactctgta gcagatgatc taacaatctg attacaatta cgccccgccc tg 52
<210> 23
   <211> 24
   <212> DNA
   <213> Artificial
<220>
   <223> primer
<400> 23
   ctgacctggc ctgctttatg catt 24
<210> 24
   <211> 26
   <212> DNA
   <213> Artificial
<220>
   <223> primer
<400> 24
   aatctggctg accgaatcgc agcaac 26
<210> 25
   <211> 650
   <212> PRT
   <213> Salmonella typhimurium
<400> 25
   Met Asn Ile Leu Gly Phe Phe Gln Arg Leu Gly Arg Ala Leu Gln Leu
   1 5 10 15
   Pro Ile Ala Val Leu Pro Val Ala Ala Leu Leu Leu Arg Phe Gly Gln
   20 25 30
   Pro Asp Leu Leu Asn Met Pro Phe Ile Ala Gln Ala Gly Gly Ser Ile
   35 40 45
   Phe Asp Asn Leu Ala Leu Val Phe Ala Ile Gly Val Ala Ser Ser Trp
   50 55 60
   Ser Lys Asp Ser Ala Gly Ala Ala Ala Leu Ala Gly Ala Val Gly Tyr
   65 70 75 80
   Phe Val Met Thr Lys Ala Met Val Thr Ile Asn Pro Glu Ile Asn Met
   85 90 95
   Gly Val Leu Ala Gly Ile Ile Thr Gly Leu Val Gly Gly Ala Val Tyr
   100 105 110
   Asn Arg Trp Ser Gly Ile Lys Leu Pro Asp Phe Leu Ser Phe Phe Gly
   115 120 125
   Gly Lys Arg Phe Val Pro Ile Ala Thr Gly Phe Phe Cys Leu Val Leu
   130 135 140
   Ala Ala Ile Phe Gly Tyr Val Trp Pro Pro Val Gln His Gly Ile His
   145 150 155 160
   Ala Gly Gly Glu Trp Ile Val Ser Ala Gly Ala Leu Gly Ser Gly Ile
   165 170 175
   Phe Gly Phe Ile Asn Arg Leu Leu Ile Pro Thr Gly Leu His Gln Val
   180 185 190
   Leu Asn Thr Ile Ala Trp Phe Gln Ile Gly Glu Phe Thr Asn Ala Ala
   195 200 205
   Gly Thr Val Phe His Gly Asp Ile Asn Arg Phe Tyr Ala Gly Asp Gly
   210 215 220
   Thr Ala Gly Met Phe Met Ser Gly Phe Phe Pro Ile Met Met Phe Gly
   225 230 235 240
   Leu Pro Gly Ala Ala Leu Ala Met Tyr Phe Ala Ala Pro Lys Glu Arg
   245 250 255
   Arg Pro Met Val Gly Gly Met Leu Leu Ser Val Ala Ile Thr Ala Phe
   260 265 270
   Leu Thr Gly Val Thr Glu Pro Leu Glu Phe Leu Phe Met Phe Leu Ala
   275 280 285
   Pro Leu Leu Tyr Leu Leu His Ala Ile Leu Thr Gly Ile Ser Leu Phe
   290 295 300
   Val Ala Thr Leu Leu Gly Ile His Ala Gly Phe Ser Phe Ser Ala Gly
   305 310 315 320
   Ala Ile Asp Tyr Val Leu Met Tyr Asn Leu Pro Ala Ala Ser Asn Asn
   325 330 335
   Val Trp Met Leu Leu Val Met Gly Val Val Phe Phe Ile Ile Tyr Phe
   340 345 350
   Leu Leu Phe Ser Ala Val Ile Arg Met Phe Asn Leu Lys Thr Pro Gly
   355 360 365
   Arg Glu Asp Lys Val Asp Glu Met Val Thr Glu Glu Ala Asn Ser Asn
   370 375 380
   Thr Glu Glu Gly Leu Thr Gln Leu Ala Thr Ser Tyr Ile Ala Ala Val
   385 390 395 400
   Gly Gly Thr Asp Asn Leu Lys Ala Ile Asp Ala Cys Ile Thr Arg Leu
   405 410 415
   Arg Leu Thr Val Asn Asp Ser Ala Arg Val Asn Asp Ala Ala Cys Lys
   420 425 430
   Arg Leu Gly Ala Ser Gly Val Val Lys Leu Asn Lys Gln Thr Ile Gln
   435 440 445
   Val Ile Val Gly Ala Lys Ala Glu Ser Ile Gly Asp Glu Met Lys Lys
   450 455 460
   Val Val Ala Arg Gly Pro Val Ala Ala Ala Ser Ala Asp Ala Ala His
   465 470 475 480
   Val Ala Thr Pro Ala Pro Ala Ala Lys Pro Gln Ala Val Pro Asn Ala
   485 490 495
   Val Thr Ile Ala Glu Leu Val Ser Pro Ile Thr Gly Glu Val Val Ala
   500 505 510
   Leu Asp Gln Val Pro Asp Glu Ala Phe Ala Ser Lys Ala Val Gly Asp
   515 520 525
   Gly Val Ala Val Lys Pro Thr Asp Lys Thr Val Val Ser Pro Ala Ala
   530 535 540
   Gly Thr Ile Val Lys Ile Phe Asn Thr Asn His Ala Phe Cys Leu Glu
   545 550 555 560
   Thr Glu Lys Gly Ala Glu Ile Val Val His Met Gly Ile Asp Thr Val
   565 570 575
   Ala Leu Asn Gly Gln Gly Phe Lys Arg Leu Val Glu Glu Gly Ala Glu
   580 585 590
   Val Thr Ala Gly Gln Pro Val Leu Glu Leu Asp Leu Asp Phe Leu Asn
   595 600 605
   Ala Asn Ala Arg Ser Met Ile Ser Pro Val Val Cys Ser Asn Ser Asp
   610 615 620
   Asp Phe Ser Ala Leu Val Ile Lys Ala Asp Gly His Val Val Ala Gly
   625 630 635 640
   Lys Thr Pro Leu Tyr Glu Ile Lys Ser Lys
   645 650
<210> 26
   <211> 650
   <212> PRT
   <213> Salmonella typhi
<400> 26
   Met Asn Ile Leu Gly Phe Phe Gln Arg Leu Gly Arg Ala Leu Gln Leu
   1 5 10 15
   Pro Ile Val Val Leu Pro Val Ala Ala Leu Leu Leu Arg Phe Gly Gln
   20 25 30
   Pro Asp Leu Leu Asn Met Pro Phe Ile Ala Gln Ala Gly Gly Ser Ile
   35 40 45
   Phe Asp Asn Leu Ala Leu Val Phe Ala Ile Gly Val Ala Ser Ser Trp
   50 55 60
   Ser Lys Asp Ser Ala Gly Ala Ala Ala Leu Ala Gly Ala Val Gly Tyr
   65 70 75 80
   Phe Val Met Thr Lys Ala Met Val Thr Ile Asn Pro Glu Ile Asn Met
   85 90 95
   Gly Val Leu Ala Gly Ile Ile Thr Gly Leu Val Gly Gly Ala Val Tyr
   100 105 110
   Asn Arg Trp Ser Gly Ile Lys Leu Pro Asp Phe Leu Ser Phe Phe Gly
   115 120 125
   Gly Lys Arg Phe Val Pro Ile Ala Thr Gly Phe Phe Cys Leu Val Leu
   130 135 140
   Ala Ala Ile Phe Gly Tyr Val Trp Pro Pro Val Gln His Gly Ile His
   145 150 155 160
   Ala Gly Gly Glu Trp Ile Val Ser Ala Gly Ala Leu Gly Ser Gly Ile
   165 170 175
   Phe Gly Phe Ile Asn Arg Leu Leu Ile Pro Thr Gly Leu His Gln Val
   180 185 190
   Leu Asn Thr Ile Ala Trp Phe Gln Ile Gly Glu Phe Thr Asn Ala Ala
   195 200 205
   Gly Thr Val Phe His Gly Asp Ile Asn Arg Phe Tyr Ala Gly Asp Gly
   210 215 220
   Thr Ala Gly Met Phe Met Ser Gly Phe Phe Pro Ile Met Met Phe Gly
   225 230 235 240
   Leu Pro Gly Ala Ala Leu Ala Met Tyr Phe Ala Ala Pro Lys Glu Arg
   245 250 255
   Arg Pro Met Val Gly Gly Met Leu Leu Ser Val Ala Ile Thr Ala Phe
   260 265 270
   Leu Thr Gly Val Thr Glu Pro Leu Glu Phe Leu Phe Met Phe Leu Ala
           275                 280                 285
   Pro Leu Leu Tyr Leu Leu His Ala Ile Leu Thr Gly Ile Ser Leu Phe
       290                 295                 300
   Val Ala Thr Leu Leu Gly Ile His Ala Gly Phe Ser Phe Ser Ala Gly
   305                 310                 315                 320
   Ala Ile Asp Tyr Val Leu Met Tyr Asn Leu Pro Ala Ala Ser Asn Asn
                   325                 330                 335
   Val Trp Met Leu Leu Val Met Gly Val Val Phe Phe Ile Ile Tyr Phe
               340                 345                 350
   Leu Leu Phe Ser Ala Val Ile Arg Met Phe Asn Leu Lys Thr Pro Gly
           355                 360                 365
   Arg Glu Asp Lys Val Asp Glu Met Val Thr Glu Glu Ala Asn Ser Asn
       370                 375                 380
   Thr Glu Glu Gly Leu Thr Gln Leu Ala Thr Ser Tyr Ile Ala Ala Val
   385                 390                 395                 400
   Gly Gly Thr Asp Asn Leu Lys Ala Val Asp Ala Cys Ile Thr Arg Leu
                   405                 410                 415
   Arg Leu Thr Val Asn Asp Ser Ala Arg Val Asn Asp Ala Ala Cys Lys
               420                 425                 430
   Arg Leu Gly Ala Ser Gly Val Val Lys Leu Asn Lys Gln Thr Ile Gln
           435                 440                 445
   Val Ile Val Gly Ala Lys Ala Glu Ser Ile Gly Asp Glu Met Lys Lys
       450                 455                 460
   Val Val Ala Arg Gly Pro Val Ala Ala Ala Ser Ala Asp Ala Ala His
   465                 470                 475                 480
   Val Ala Thr Pro Ala Pro Ala Ala Lys Pro Gln Ala Val Pro Asn Ala
                   485                 490                 495
   Val Thr Ile Ala Glu Leu Val Ser Pro Ile Thr Gly Glu Val Val Ala
               500                 505                 510
   Leu Asp Gln Val Pro Asp Glu Ala Phe Ala Ser Lys Ala Val Gly Asp
           515                 520                 525
   Gly Val Ala Val Lys Pro Thr Asp Lys Thr Val Val Ser Pro Ala Ala
       530                 535                 540
   Gly Thr Ile Val Lys Ile Phe Asn Thr Asn His Ala Phe Cys Leu Glu
   545                 550                 555                 560
   Thr Glu Lys Gly Ala Glu Ile Val Val His Met Gly Ile Asp Thr Val
                   565                 570                 575
   Ala Leu Asn Gly Gln Gly Phe Lys Arg Leu Val Glu Glu Gly Ala Glu
               580                 585                 590
   Val Thr Ala Gly Gln Pro Val Leu Glu Leu Asp Leu Asp Phe Leu Asn
   595 600 605
   Ala Asn Ala Arg Ser Lys Ile Ser Pro Val Val Cys Ser Asn Ser Asp
   610 615 620
   Asp Phe Ser Ala Leu Val Ile Lys Ala Asp Gly His Val Val Ala Gly
   625 630 635 640
   Gln Thr Pro Leu Tyr Glu Ile Lys Ser Lys
   645 650
<210> 27
   <211> 648
   <212> PRT
   <213> Shigella flexneri
<400> 27
   Met Asn Ile Leu Gly Phe Phe Gln Arg Leu Gly Arg Ala Leu Gln Leu
   1               5                   10                  15
   Pro Ile Ala Val Leu Pro Val Ala Ala Leu Leu Leu Arg Phe Gly Gln
               20                  25                  30
   Pro Asp Leu Leu Asn Val Ala Phe Ile Ala Gln Ala Gly Gly Ala Ile
           35                  40                  45
   Phe Asp Asn Leu Ala Leu Ile Phe Ala Ile Gly Val Ala Ser Ser Trp
       50                  55                  60
   Ser Lys Asp Ser Ala Gly Ala Ala Ala Leu Ala Gly Ala Val Gly Tyr
   65                  70                  75                  80
   Phe Val Leu Thr Lys Ala Met Val Thr Ile Asn Pro Glu Ile Asn Met
                   85                  90                  95
   Gly Val Leu Ala Gly Ile Ile Thr Gly Leu Val Gly Gly Ala Ala Tyr
               100                 105                 110
   Asn Arg Trp Ser Asp Ile Lys Leu Pro Asp Phe Leu Ser Phe Phe Gly
           115                 120                 125
   Gly Lys Arg Phe Val Pro Ile Ala Thr Gly Phe Phe Cys Leu Val Leu
       130                 135                 140
   Ala Ala Ile Phe Gly Tyr Val Trp Pro Pro Val Gln His Ala Ile His
   145                 150                 155                 160
   Ala Gly Gly Glu Trp Ile Val Ser Ala Gly Ala Leu Gly Ser Gly Ile
                   165                 170                 175
   Phe Gly Phe Ile Asn Arg Leu Leu Ile Pro Thr Gly Leu His Gln Val
               180                 185                 190
   Leu Asn Thr Ile Ala Trp Phe Gln Ile Gly Glu Phe Thr Asn Ala Ala
           195                 200                 205
   Gly Thr Val Phe His Gly Asp Ile Asn Arg Phe Tyr Ala Gly Asp Gly
       210                 215                 220
   Thr Ala Gly Met Phe Met Ser Gly Phe Phe Pro Ile Met Met Phe Gly
   225 230 235 240
   Leu Pro Gly Ala Ala Leu Ala Met Tyr Phe Ala Ala Pro Lys Glu Arg
   245 250 255
   Arg Pro Met Val Gly Gly Met Leu Leu Ser Val Ala Val Thr Ala Phe
   260 265 270
   Leu Thr Gly Val Thr Glu Pro Leu Glu Phe Leu Phe Met Phe Leu Ala
   275 280 285
   Pro Leu Leu Tyr Leu Leu His Ala Leu Leu Thr Gly Ile Ser Leu Phe
   290 295 300
   Val Ala Thr Leu Leu Gly Ile His Ala Gly Phe Ser Phe Ser Ala Gly
   305 310 315 320
   Ala Ile Asp Tyr Ala Leu Met Tyr Asn Leu Pro Ala Ala Ser Gln Asn
   325 330 335
   Val Trp Met Leu Leu Val Met Gly Val Ile Phe Phe Ala Ile Tyr Phe
   340 345 350
   Val Val Phe Ser Leu Val Ile Arg Met Phe Asn Leu Lys Thr Pro Gly
   355 360 365
   Arg Glu Asp Lys Glu Asp Glu Ile Val Thr Glu Glu Ala Asn Ser Asn
   370 375 380
   Thr Asp Ser Cys Leu Thr Gln Leu Ala Thr Asn Tyr Ile Ala Ala Val
   385 390 395 400
   Gly Gly Thr Asp Asn Leu Lys Ala Ile Asp Ala Cys Ile Thr Arg Leu
   405 410 415
   Arg Leu Thr Val Ala Asp Ser Ala Arg Val Asn Asp Thr Met Cys Lys
   420 425 430
   Arg Leu Gly Ala Ser Gly Val Val Lys Leu Asn Lys Gln Thr Ile Gln
   435 440 445
   Val Ile Val Gly Ala Lys Ala Glu Ser Ile Gly Asp Ala Met Lys Lys
   450 455 460
   Val Val Ala Arg Gly Pro Val Ala Ala Ala Ser Ala Glu Ala Thr Pro
   465 470 475 480
   Ala Thr Ala Ala Pro Val Ala Lys Pro Gln Ala Val Pro Asn Ala Val
   485 490 495
   Ser Ile Ala Glu Leu Val Ser Pro Ile Thr Gly Asp Val Val Ala Leu
   500 505 510
   Asp Gln Val Pro Asp Glu Ala Phe Ala Ser Lys Ala Val Gly Asp Gly
   515 520 525
   Val Ala Val Lys Pro Thr Asp Lys Ile Val Val Ser Pro Ala Ala Gly
   530 535 540
   Thr Ile Val Lys Ile Phe Asn Thr Asn His Ala Phe Cys Leu Glu Thr
   545 550 555 560
   Glu Lys Gly Ala Glu Ile Val Val His Met Gly Ile Asp Thr Val Ala
   565 570 575
   Leu Glu Gly Lys Gly Phe Lys Arg Leu Val Glu Glu Gly Ala Gln Val
   580 585 590
   Ser Ala Gly Gln Pro Ile Leu Glu Met Asp Leu Asp Tyr Leu Asn Ala
   595 600 605
   Asn Ala Arg Ser Met Ile Ser Pro Val Val Cys Ser Asn Ile Asp Asp
   610 615 620
   Phe Ser Gly Leu Leu Ile Lys Ala Gln Gly His Val Val Ala Gly Gln
   625 630 635 640
   Thr Pro Leu Tyr Glu Ile Lys Lys
   645
<210> 28
   <211> 651
   <212> PRT
   <213> Klebsiella pneumoniae
<400> 28
   Met Asn Ile Leu Gly Phe Phe Gln Arg Leu Gly Arg Ala Leu Gln Leu
   1 5 10 15
   Pro Ile Ala Val Leu Pro Val Ala Ala Leu Leu Leu Arg Phe Gly Gln
   20 25 30
   Pro Asp Leu Leu Asn Val Pro Phe Ile Ala Gln Ala Gly Gly Ala Ile
   35 40 45
   Phe Asp Asn Leu Ala Leu Ile Phe Ala Ile Gly Val Ala Ser Ser Trp
   50 55 60
   Ser Lys Asp Asn Ala Gly Ser Ala Ala Leu Ala Gly Ala Val Gly Tyr
   65 70 75 80
   Phe Val Met Thr Lys Ala Met Val Thr Ile Asn Pro Glu Ile Asn Met
   85 90 95
   Gly Val Leu Ala Gly Ile Ile Thr Gly Leu Val Ala Gly Ala Val Tyr
   100 105 110
   Asn Arg Trp Ala Gly Ile Lys Leu Pro Asp Phe Leu Ser Phe Phe Gly
   115 120 125
   Gly Lys Arg Phe Val Pro Ile Ala Thr Gly Phe Phe Cys Leu Ile Leu
   130 135 140
   Ala Ala Ile Phe Gly Tyr Val Trp Pro Pro Val Gln His Ala Ile His
   145 150 155 160
   Ser Gly Gly Glu Trp Ile Val Ser Ala Gly Ala Leu Gly Ser Gly Ile
   165 170 175
   Phe Gly Phe Ile Asn Arg Leu Leu Ile Pro Thr Gly Leu His Gln Val
               180                 185                 190
   Leu Asn Thr Ile Ala Trp Phe Gln Ile Gly Glu Phe Thr Asn Ala Ala
           195                 200                 205
   Gly Thr Val Phe His Gly Asp Ile Asn Arg Phe Tyr Ala Gly Asp Gly
       210                 215                 220
   Thr Ala Gly Met Phe Met Ser Gly Phe Phe Pro Ile Met Met Phe Gly
   225                 230                 235                 240
   Leu Pro Gly Ala Ala Leu Ala Met Tyr Leu Ala Ala Pro Lys Ala Arg
                   245                 250                 255
   Arg Pro Met Val Gly Gly Met Leu Leu Ser Val Ala Ile Thr Ala Phe
               260                 265                 270
   Leu Thr Gly Val Thr Glu Pro Leu Glu Phe Leu Phe Leu Phe Leu Ala
           275                 280                 285
   Pro Leu Leu Tyr Leu Leu His Ala Val Leu Thr Gly Ile Ser Leu Phe
       290                 295                 300
   Ile Ala Thr Ala Leu Gly Ile His Ala Gly Phe Ser Phe Ser Ala Gly
   305                 310                 315                 320
   Ala Ile Asp Tyr Val Leu Met Tyr Ser Leu Pro Ala Ala Ser Lys Asn
                   325                 330                 335
   Val Trp Met Leu Leu Val Met Gly Val Val Phe Phe Phe Val Tyr Phe
               340                 345                 350
   Leu Leu Phe Ser Ala Val Ile Arg Met Phe Asn Leu Lys Thr Pro Gly
           355                 360                 365
   Arg Glu Asp Lys Ala Ala Asp Val Val Thr Glu Glu Ala Asn Ser Asn
       370                 375                 380
   Thr Glu Glu Gly Leu Thr Gln Leu Ala Thr Ser Tyr Ile Ala Ala Val
   385                 390                 395                 400
   Gly Gly Thr Asp Asn Leu Lys Ala Ile Asp Ala Cys Ile Thr Arg Leu
                   405                 410                 415
   Arg Leu Thr Val Gly Asp Ser Ala Lys Val Asn Asp Ala Ala Cys Lys
               420                 425                 430
   Arg Leu Gly Ala Ser Gly Val Val Lys Leu Asn Lys Gln Thr Ile Gln
           435                 440                 445
   Val Ile Val Gly Ala Lys Ala Glu Ser Ile Gly Asp Glu Met Lys Lys
       450                 455                 460
   Val Val Thr Arg Gly Pro Val Ala Ala Ala Ala Ala Ala Pro Ala Gly
   465                 470                 475                 480
   Asn Val Ala Thr Ala Ala Pro Ala Ala Lys Pro Gln Ala Val Ala Asn
                   485                 490                 495
   Ala Lys Thr Val Glu Ser Leu Val Ser Pro Ile Thr Gly Asp Val Val
   500 505 510
   Ala Leu Glu Gln Val Pro Asp Glu Ala Phe Ala Ser Lys Ala Val Gly
   515 520 525
   Asp Gly Ile Ala Val Lys Pro Thr Asp Asn Ile Val Val Ala Pro Ala
   530 535 540
   Ala Gly Thr Val Val Lys Ile Phe Asn Thr Asn His Ala Phe Cys Leu
   545 550 555 560
   Glu Thr Asn Asn Gly Ala Glu Ile Val Val His Met Gly Ile Asp Thr
   565 570 575
   Val Ala Leu Glu Gly Lys Gly Phe Lys Arg Leu Val Glu Glu Gly Thr
   580 585 590
   Asp Val Lys Ala Gly Glu Pro Ile Leu Glu Met Asp Leu Asp Phe Leu
   595 600 605
   Asn Ala Asn Ala Arg Ser Met Ile Ser Pro Val Val Cys Ser Asn Ser
   610 615 620
   Asp Asp Tyr Ser Ala Leu Val Ile Leu Ala Ser Gly Lys Val Val Ala
   625 630 635 640
   Gly Gln Thr Pro Leu Tyr Glu Ile Lys Gly Lys
   645 650
<210> 29
   <211> 677
   <212> PRT
   <213> Yersinis pestis
<400> 29
   Met Ser Ile Leu Gly Tyr Leu Gln Lys Val Gly Arg Ala Leu Met Val
   1 5 10 15
   Pro Val Ala Thr Leu Pro Ala Ala Ala Ile Leu Met Gly Val Gly Tyr
   20 25 30
   Trp Ile Asp Pro Val Gly Trp Gly Ala Asp Asn Ala Leu Ala Ala Leu
   35 40 45
   Phe Ile Lys Ser Gly Ala Ala Ile Ile Glu Asn Met Ser Val Leu Phe
   50 55 60
   Ala Ile Gly Val Ala Tyr Gly Met Ser Lys Asp Lys Asp Gly Ala Ala
   65 70 75 80
   Ala Leu Thr Gly Phe Val Gly Phe Leu Val Leu Thr Thr Leu Cys Ser
   85 90 95
   Pro Ala Ala Val Ser Met Ile Lys Gln Ile Pro Leu Asp Gln Val Pro
   100 105 110
   Ala Ala Phe Gly Lys Ile Glu Asn Gln Phe Val Gly Ile Leu Val Gly
   115 120 125
   Ile Ile Ser Ala Glu Leu Tyr Asn Arg Phe Ser Gly Val Glu Leu Pro
           130                  135                  140
   Lys Ala Leu Ser Phe Phe Ser Gly Arg Arg Leu Val Pro Ile Leu Thr
   145                 150                 155                 160
   Ser Phe Leu Met Ile Ala Val Ala Phe Met Leu Met Tyr Ile Trp Pro
                   165                 170                 175
   Leu Ile Tyr Asn Ala Leu Val Thr Phe Gly Glu Tyr Ile Lys Asp Leu
               180                 185                 190
   Gly Ser Val Gly Ala Gly Ile Tyr Ala Phe Phe Asn Arg Leu Leu Ile
           195                 200                 205
   Pro Val Gly Leu His His Ala Leu Asn Ser Val Phe Trp Phe Asp Val
       210                 215                 220
   Ala Gly Ile Asn Asp Ile Pro Asn Phe Leu Gly Gly Gln Glu Ser Ile
   225                 230                 235                 240
   Asn Lys Gly Thr Gly Ile Val Gly Ile Thr Gly Arg Tyr Gln Ala Gly
                   245                 250                 255
   Phe Phe Pro Ile Met Met Phe Gly Leu Pro Gly Ala Ala Leu Ala Ile
               260                 265                 270
   Tyr His Cys Ala Arg Pro Glu Asn Lys Ala Lys Val Ala Gly Ile Met
           275                 280                 285
   Met Ala Gly Ala Phe Ala Ala Phe Phe Thr Gly Ile Thr Glu Pro Leu
       290                 295                 300
   Glu Phe Ser Phe Met Phe Val Ala Pro Val Leu Tyr Phe Leu His Ala
   305                 310                 315                 320
   Val Leu Thr Gly Ile Ser Val Phe Ile Ala Ala Ser Met His Trp Ile
                   325                 330                 335
   Ala Gly Phe Gly Phe Ser Ala Gly Leu Val Asp Met Val Leu Ser Ser
               340                 345                 350
   Arg Asn Pro Leu Ala Thr Gln Trp Tyr Met Leu Ile Pro Gln Gly Leu
           355                 360                 365
   Ile Phe Phe Val Ile Tyr Tyr Leu Val Phe Arg Phe Thr Ile Gln Lys
       370                 375                 380
   Phe Asn Leu Leu Thr Pro Gly Arg Glu Leu Ala Val Glu Gly Ser Glu
   385                 390                 395                 400
   Glu Asp Gly Tyr Asp Val Asn Val Asp Lys Thr Pro Ala Val Asn Glu
                   405                 410                 415
   Ser Glu Ile Asn Ser Leu Ala Arg Arg Tyr Ile Gly Ala Ile Gly Gly
               420                 425                 430
   Ser Asp Asn Leu Thr Ala Ile Asp Ala Cys Ile Thr Arg Leu Arg Leu
           435                 440                 445
   Asn Val Lys Asp Ser Ala Leu Val Asn Asp Ser Val Ala Lys Arg Leu
   450 455 460
   Gly Ala Ser Gly Val Ile Arg Leu Asn Lys Gln Ser Val Gln Ile Ile
   465 470 475 480
   Val Gly Thr Arg Ala Glu Leu Ile Ala Ala Ala Met Arg Thr Val Leu
   485 490 495
   Ala Gly Gly Pro Ile Pro Ala Ala Ser Ser Asn Ala Ala Pro Thr Gly
   500 505 510
   Ala Arg Pro Gln Ala Val Ile Asn Thr Ala Lys Thr Ala Ser Leu Val
   515 520 525
   Leu Val Ser Pro Ile Thr Gly Asp Val Val Pro Leu Ala Gln Val Pro
   530 535 540
   Asp Glu Ala Phe Ala Ser Lys Ala Val Gly Glu Gly Val Ala Ile Arg
   545 550 555 560
   Pro Thr Asp Lys Ile Val Val Ser Pro Ala Ser Gly Thr Ile Val Lys
   565 570 575
   Ile Phe Asn Thr Asp His Ala Phe Cys Leu Glu Thr Glu Thr Gly Ala
   580 585 590
   Glu Ile Val Val His Ile Gly Ile Asp Thr Val Lys Leu Asn Gly Gln
   595 600 605
   Gly Phe Thr Arg Leu Val Glu Glu Gly Thr Thr Val Val Ala Gly Gln
   610 615 620
   Pro Val Leu Glu Leu Asp Leu Ala Tyr Leu Asn Ala Asn Ala His Ser
   625 630 635 640
   Met Ile Ser Pro Val Val Val Ser Asn Ile Asp Asp Tyr Ala Gly Ile
   645 650 655
   Ser Leu Leu Ala Ser Gly Ser Val Val Ala Gly Gln Ser Gln Leu Phe
   660 665 670
   Glu Ile Arg Gly Lys
   675
<210> 30
   <211> 677
   <212> PRT
   <213> Yersinis pseudotuberculosis
<400> 30
   Met Ser Ile Leu Gly Tyr Leu Gln Lys Val Gly Arg Ala Leu Met Val
   1               5                   10                  15
   Pro Val Ala Thr Leu Pro Ala Ala Ala Ile Leu Met Gly Val Gly Tyr
               20                  25                  30
   Trp Ile Asp Pro Val Gly Trp Gly Ala Asp Asn Ala Leu Ala Ala Leu
           35                  40                  45
   Phe Ile Lys Ser Gly Ala Ala Ile Ile Glu Asn Met Ser Val Leu Phe
       50                  55                  60
   Ala Ile Gly Val Ala Tyr Gly Met Ser Lys Asp Lys Asp Gly Ala Ala
   65                  70                  75                  80
   Ala Leu Thr Gly Phe Val Gly Phe Leu Val Leu Thr Thr Leu Cys Ser
                   85                  90                  95
   Pro Ala Ala Val Ser Met Ile Lys Gln Ile Pro Leu Asp Gln Val Pro
               100                 105                 110
   Ala Ala Phe Gly Lys Ile Glu Asn Gln Phe Val Gly Ile Leu Val Gly
           115                 120                 125
   Ile Ile Ser Ala Glu Leu Tyr Asn Arg Phe Ser Gly Val Glu Leu Pro
       130                 135                 140
   Lys Ala Leu Ser Phe Phe Ser Gly Arg Arg Leu Val Pro Ile Leu Thr
   145                 150                 155                 160
   Ser Phe Leu Met Ile Ala Val Ala Phe Met Leu Met Tyr Ile Trp Pro
                   165                 170                 175
   Leu Ile Tyr Asn Ala Leu Val Thr Phe Gly Glu Tyr Ile Lys Asp Leu
               180                 185                 190
   Gly Ser Val Gly Ala Gly Ile Tyr Ala Phe Phe Asn Arg Leu Leu Ile
           195                 200                 205
   Pro Val Gly Leu His His Ala Leu Asn Ser Val Phe Trp Phe Asp Val
       210                 215                 220
   Ala Gly Ile Asn Asp Ile Pro Asn Phe Leu Gly Gly Gln Glu Ser Ile
   225                 230                 235                 240
   Asn Lys Gly Thr Gly Ile Val Gly Ile Thr Gly Arg Tyr Gln Ala Gly
                   245                 250                 255
   Phe Phe Pro Ile Met Met Phe Gly Leu Pro Gly Ala Ala Leu Ala Ile
               260                 265                 270
   Tyr His Cys Ala Arg Pro Glu Asn Lys Ala Lys Val Ala Gly Ile Met
           275                 280                 285
   Met Ala Gly Ala Phe Ala Ala Phe Phe Thr Gly Ile Thr Glu Pro Leu
       290                 295                 300
   Glu Phe Ser Phe Met Phe Val Ala Pro Val Leu Tyr Phe Leu His Ala
   305                 310                 315                 320
   Val Leu Thr Gly Ile Ser Val Phe Ile Ala Ala Ser Met His Trp Ile
                   325                 330                 335
   Ala Gly Phe Gly Phe Ser Ala Gly Leu Val Asp Met Val Leu Ser Ser
               340                 345                 350
   Arg Asn Pro Leu Ala Thr Gln Trp Tyr Met Leu Ile Pro Gln Gly Leu
           355                 360                 365
   Ile Phe Phe Val Ile Tyr Tyr Leu Val Phe Arg Phe Thr Ile Gln Lys
           370                 375                 380
   Phe Asn Leu Leu Thr Pro Gly Arg Glu Leu Ala Val Glu Gly Ser Glu
   385                 390                 395                 400
   Glu Asp Gly Tyr Asp Val Asn Val Asp Lys Thr Pro Ala Val Asn Glu
                   405                 410                 415
   Ser Glu Ile Asn Gly Leu Ala Arg Arg Tyr Ile Gly Ala Ile Gly Gly
               420                 425                 430
   Ser Asp Asn Leu Thr Ala Ile Asp Ala Cys Ile Thr Arg Leu Arg Leu
           435                 440                 445
   Asn Val Lys Asp Ser Ala Leu Val Asn Asp Ser Val Ala Lys Arg Leu
       450                 455                 460
   Gly Ala Ser Gly Val Ile Arg Leu Asn Lys Gln Ser Val Gln Ile Ile
   465                 470                 475                 480
   Val Gly Thr Arg Ala Glu Leu Ile Ala Ala Ala Met Arg Thr Val Leu
                   485                 490                 495
   Ala Gly Gly Pro Ile Pro Ala Ala Ser Ser Asn Ala Ala Pro Thr Gly
               500                 505                 510
   Ala Arg Pro Gln Ala Val Ile Asn Thr Ala Lys Thr Ala Ser Leu Val
           515                 520                 525
   Leu Val Ser Pro Ile Thr Gly Asp Val Val Pro Leu Ala Gln Val Pro
       530                 535                 540
   Asp Glu Ala Phe Ala Ser Lys Ala Val Gly Glu Gly Val Ala Ile Arg
   545                 550                 555                 560
   Pro Thr Asp Lys Ile Val Val Ser Pro Ala Ser Gly Thr Ile Val Lys
                   565                 570                 575
   Ile Phe Asn Thr Asp His Ala Phe Cys Leu Glu Thr Glu Thr Gly Ala
               580                 585                 590
   Glu Ile Val Val His Ile Gly Ile Asp Thr Val Lys Leu Asn Gly Gln
           595                 600                 605
   Gly Phe Thr Arg Leu Val Glu Glu Gly Thr Thr Val Val Ala Gly Gln
       610                 615                 620
   Pro Val Leu Glu Leu Asp Leu Ala Tyr Leu Asn Ala Asn Ala His Ser
   625                 630                 635                 640
   Met Ile Ser Pro Val Val Val Ser Asn Ile Asp Asp Tyr Ala Gly Ile
                   645                 650                 655
   Ser Leu Leu Ala Ser Gly Ser Val Val Ala Gly Gln Ser Gln Leu Phe
               660                 665                 670
   Glu Ile Arg Gly Lys
           675

## Description

### Technical field

The present invention relates to a method for producing an L-amino acid by fermentation, and more specifically to genes which aid in this fermentation. These genes are useful for the improving L-amino acid production, for example, for production of L-threonine, L-lysine, L-histidine, L-phenylalanine, L-arginine, L-tryptophan, and L-glutamic acid.

### Background art

Conventionally, L-amino acids are industrially produced by fermentation methods utilizing strains of microorganisms obtained from natural sources, or mutants thereof. Typically, the microorganisms are modified to enhance production yields of L-amino acids.

Many techniques to enhance L-amino acid production yields have been reported, including transformation of microorganisms with recombinant DNA (see, for example, U.S. Patent No. 4,278,765). Other techniques for enhancing production yields include increasing the activities of enzymes involved in amino acid biosynthesis and/or desensitizing the target enzymes of the feedback inhibition by the resulting L-amino acid (see, for example, WO 95/16042 or U.S. Patent Nos. 4,346,170, 5,661,012 and 6,040,160).

Strains useful in production of L-threonine by fermentation are known, including strains with increased activities of enzymes involved in L-threonine biosynthesis (U.S. Patent Nos. 5,175,107; 5,661,012; 5,705,371; 5,939,307; EP 0219027), strains resistant to chemicals such as L-threonine and its analogs (WO 01/14525A1, EP 301572 A2, U.S. Patent No. 5,376,538), strains with target enzymes desensitized to feedback inhibition by the produced L-amino acid or its by-products (U.S. Patent Nos. 5,175,107; 5,661,012), and strains with inactivated threonine degradation enzymes (U.S. Patent Nos. 5,939,307; 6,297,031).

The known threonine-producing strain *Escherichia coli* VKPM B-3996 (U.S. Patent Nos. 5,175,107 and 5,705,371) is presently one of the best known threonine producers. To construct the VKPM B-3996 strain, several mutations and a plasmid, described below, were introduced into the parent strain *E. coli* K-12 (VKPM B-7). A mutant *thrA* gene (mutation *thrA442*) encodes aspartokinase homoserine dehydrogenase I, which is resistant to feedback inhibition by threonine. A mutant *ilvA* gene (mutation *ilvA*442) encodes threonine deaminase which has a decreased activity, and results in a decreased rate of isoleucine biosynthesis and a leaky phenotype of isoleucine starvation. In bacteria containing the *ilvA*442 mutation, transcription of the *thrABC* operon is not repressed by isoleucine; and therefore, these strains are very efficient for threonine production. Inactivation of the *tdh* gene encoding threoninedehydrogenase results in prevention of threonine degradation. The genetic determinant of saccharose assimilation (*scrKYABR* genes) was transferred to said strain. To increase expression of the genes controlling threonine biosynthesis, the plasmid pVIC40 containing the mutant threonine operon *thrA442BC* was introduced into the intermediate strain TDH6. The amount of L-threonine which accumulates during fermentation of the strain can be up to 85 g/l.

By optimizing the main biosynthetic pathway of a desired compound, further improvement of L-amino acid producing strains can be accomplished via supplementation of the bacterium with increasing amounts of sugars as a carbon source, for example, glucose. Despite the efficiency of glucose transport by PTS, access to the carbon source in a highly productive strain still may be insufficient.

It is known that the active transport of sugars and other metabolites into bacterial cells is accomplished by several transport systems.

The divergent *nagE-BACD* operon encodes proteins involved in the uptake and degradation of N-acetylglucosamine (GlcNAc). The *nagE* gene encodes the N-acetylglucosamine-specific transporter of the phosphotransferase system (PTS) involved in the uptake of GlcNAc and producing intracellular GlcNAc-6-P, while the *nagBACD* genes encode the two enzymes that degrade GlcNAc-6-P into fructose-6-P (glucosamine-P deaminase (NagB) and N-acetylglucosamine-6-P deacetylase (NagA)), the *nag* regulon transcriptional regulator (NagC) and a gene of unknown function which is, however, homologous to functionally characterized phosphatases (NagD) (Potsma, P.W., Lengeler, J.W. and Jacobson, G.R. Chapter 72. In Esclaerichia coli and Salmonella, Second Edition, Editor in Chief: F.C. Neidhardt, ASM Press, Washington D.C., 1996). The Nag repressor, encoded by the *nagC* gene, binds to two operators, which overlap the *nagE* and *nagB* promoters and forms a repression loop of DNA (Plumbridge, J. and Kolb, A. Nucleic Acids Res., 26(5), 1254-60 (1998)). Expression of both *nagE* and *nagB* is stimulated by the cAMP/CAP complex, the effect being particularly pronounced for *nagE*. A binding site for cAMP/CAP is located between the two promoters, i.e. it lies within the DNA which forms a loop. The two arms of the *nag* regulon are induced in parallel by GlcNAc and glucosamine (GlcN), but the growth and induction characteristics of GlcNAc and GlcN are very different (Plumbridge, J.A., J. Bacteriol., 172, 2728-2735 (1990)). There is little information about the uptake of other sugars by NagE.

The Enzyme II (II^{Nag}), encoded by the *nagE* gene, contains the three functional domains IICBA^{Nag} fused in one polypeptide. GlcNAc is transported via the EIIC, and phosphate is subsequently transferred from EIIA via the EIIB component onto the C6-hydroxyl group of the imported GlcNAc molecule. EIIA itself is phosphorylated by PEP, and involves the general enzymes of the PTS cascade (EI and HPr). Therefore the NagE can replace Crr (catabolite repression regulator) in Crr-dependent carbohydrate transport and phosphorylation (Potsma, P.W., Lengeler, J.W. and Jacobson, G.R. Chapter 72. In Escherichia coli and Salmonella, Second Edition, Editor in Chief: F.C. Neidhardt, ASM Press, Washington D.C., 1996).

A method to produce glucosamine by fermentation using a microorganism with the increased activity of glucosamine-6-phosphate synthase has been disclosed (U.S. Patent No. 6,372,457), wherein an additional activity of at least one protein from the group containing N-acetylglucosamine-specific enzyme II^{Nag} (N-acetylglucosamine permease) encoded by the *nagE* gene is decreased.

In many bacteria, components of the phosphoenolpyruvate-dependent sugar phosphotransferase system (PTS) trigger C-regulation by mechanisms known as carbon catabolite repression and inducer exclusion (Potsma, P.W., Lengeler, J.W. and Jacobson, G.R. Chapter 75 and Saier, M. H., Jr., Ramseier, T. M., and Reizer, J. Chapter. In Escherichia coli and Salmonella, Second Edition, Editor in Chief: F.C. Neidhardt, ASM Press, Washington D.C., 1996). One key element of PTS system in *Escherichia coli* is the enzyme IIA^{Glucose} (IIA^{Glc}) encoded by the *crr* gene, a small hydrophilic protein which has, in addition to its transport function, a central regulatory role in carbon catabolite-repression and inducer exclusion. IIA^{Glc} is phosphorylated by the general PTS proteins, which are histidine-containing phosphoryl carrier protein (HPr) and enzyme I (EI). In turn, it phosphorylates the sugar-specific PTS permeases that catalyse the uptake of glucose, trehalose, and sucrose (Postma, P.W., Lengeler, J.W. & Jacobson, G.R., Microbiol. Rev., 57, 543-594 (1993)). The unphosphorylated IIA^{Glc} inhibits a set of catabolic enzymes and sugar permeases by protein-protein interaction (inducer exclusion). At the same time the cellular cAMP level is low, because dephosphorylated IIA^{Glc} is unable to stimulate adenylate cyclase. Under these conditions the cAMP-dependent catabolite activator protein CAP, which serves as a global activator of many catabolite-controlled genes, remains in a switched off state. IIA^{Glc} further appears to be involved in carbon catabolite repression exerted by non-PTS substrates such as glucose 6-phosphate (Hogema, B.M.et al, Mol. Microbiol., 28, 755-765 (1998)). This could be correlated with the variation of the phosphorylation state of IIA^{Glc}.

Recently, another cellular function of IIA^{Glc} has been proposed. It is suggested that it may be involved in the linkage between carbon metabolism and stress response (Ueguchi, C., Misonou, N. & Mizuno, T., J. Bacteriol.,183, 520-527 (2001)). Mutations in the *crr* gene, which encodes IIA^{Glc}, exhibit a pleiotropic catabolite repression resistant phenotype.

A method for producing a target substance, such as L-lysine, L-threonine and L-phenylalanine, utilizing a microorganism having mutation in the *crr* gene was disclosed (EP1254957A2). Process for the fermentative preparation of L-amino acids, in particular L-threonine, using microorganisms of the *Enterobacteriaceae* family in which one or more of the genes chosen from the group consisting of *dps, hns, lrp, pgm, fba, ptsG, ptsH, ptsI, crr, mopB, ahpC* and *ahpF,* or nucleotide sequences which code for these, is (are) attenuated, in particular eliminated, was also disclosed in WO03004662A2 without any working examples.

Plumbridge JA, 1991, discloses the nag regulon, and null mutations in each of its genes by insertion of antibiotic resistance cartridges.

However, there have been no reports to date of using a bacterium of the *Enterobacteriaceae family* having an enhanced activity of N-acetylglucosamine PTS permease or a bacterium of the *Enterobacteriaceae family* having an enhanced activity of N-acetylglucosamine PTS permease and an inactivated *crr* gene for increasing the production of L-amino acids.

### Disclosure of the Invention

Objects of the present invention include enhancing the productivity of L-amino acid-producing strains and providing a method for producing non-aromatic or aromatic L-amino acids using these strains.

The above objects were achieved by finding that increasing the expression of the *nagE* gene encoding N-acetylglucosamine permease can enhance production of L-amino acids, such as L-threonine, L-lysine, L-histidine, L-phenylalanine, L-arginine, L-tryptophan, and L-glutamic acid.

It is an object of the present disclosure to provide an L-amino acid-producing bacterium of the *Enterobacteriaceae* family, wherein said bacterium has been modified to enhance an activity of N-acetylglucosamine permease.

It is a further object of the present disclosure to provide the bacterium described above, wherein the activity of N-acetylglucosamine permease is enhanced by increasing the expression of a gene which encodes N-acetylglucosamine permease.

It is a further object of the present disclosure to provide the bacterium described above, wherein the activity of N-acetylglucosamine permease is enhanced by modifying an expression control sequence of the gene encoding N-acetylglucosamine permease so that the gene expression is enhanced or by increasing the copy number of the gene encoding N-acetylglucosamine permease.

It is a further object of the present disclosure to provide the bacterium described above, wherein said bacterium has been additionally modified to enhance an activity of glucokinase.

It is a further object of the present disclosure to provide the bacterium described above, where said bacterium has been additionally modified to enhance an activity of xylose isomerase.

It is a further object of the present disclosure to provide the bacterium described above, wherein said bacterium is selected from the group consisting of the genera *Escherichia, Enterobacter, Erwinia, Klebsiella, Pantoea, Providencia, Salmonella, Serratia, Shigella* and *Morganella.*

It is a further object of the present disclosure to provide the bacterium described above, wherein said gene encodes an N-acetylglucosamine permease selected from the group consisting of:
(A) a protein which comprises the amino acid sequence of SEQ ID NO: 2, and
(B) a variant protein of the amino acid sequence shown in SEQ ID NO: 2, and which has an activity of N-acetylglucosamine permease.

It is a further object of the present disclosure to provide the bacterium described above, wherein said gene encoding N-acetylglucosamine permease comprises a DNA selected from the group consisting of:
(a) a DNA which comprises a nucleotide sequence of nucleotides 1 to 1947 in SEQ ID NO: 1, and
(b) a DNA which is hybridizable with a nucleotide sequence of nucleotides 1-1947 in SEQ ID NO: 1, or a probe which can be prepared from said nucleotide sequence under stringent conditions, and encodes a protein having an activity of N-acetylglucosamine permease.

It is a further object of the present disclosure to provide the bacterium described above, wherein said stringent conditions comprise those in which washing is performed at 60°C at a salt concentration of 1 x SSC and 0.1 % SDS, for approximately 15 minutes.

It is a further object of the present disclosure to provide the bacterium described above, wherein said bacterium is an L-threonine producing bacterium.

It is a further object of the present disclosure to provide the bacterium described above, wherein said bacterium has been additionally modified to enhance expression of one or more of the genes selected from the group consisting of
- the mutant *thrA* gene which codes for aspartokinase homoserine dehydrogenase I and is resistant to feedback inhibition by threonine;
- the *thrB* gene which codes for homoserine kinase;
- the *thrC* gene which codes for threonine synthase;
- the *rhtA* gene which codes for a putative transmembrane protein;
- the *asd* gene which codes for aspartate-β-semialdehyde dehydrogenase; and
- the *aspC* gene which codes for aspartate aminotransferase (aspartate transaminase).

It is a further object of the present disclosure to provide the bacterium described above, wherein said bacterium has been additionally modified so that the *crr* gene which codes for catabolite repression regulator is inactivated.

It is a further object of the present disclosure to provide the bacterium described above, wherein said bacterium is an L-lysine producing bacterium.

It is a further object of the present disclosure to provide the bacterium described above, wherein said bacterium is an L-histidine producing bacterium.

It is a further object of the present disclosure to provide the bacterium described above, wherein said bacterium is an L-phenylalanine producing bacterium.

It is a further object of the present disclosure to provide the bacterium described above, wherein said bacterium is an L-arginine producing bacterium.

It is a further object of the present disclosure to provide the bacterium described above, wherein said bacterium is an L-tryptophan producing bacterium.

It is a further object of the present disclosure to provide the bacterium described above, wherein said bacterium is an L-glutamic acid producing bacterium.

It is a further object of the present invention to provide a method for producing an L-amino acid which comprises cultivating the bacterium described above in a culture medium, allowing accumulation of the L-amino acid in the culture medium, and isolating the L-amino acid from the culture medium.

It is a further object of the present invention to provide the method described above, wherein said L-amino acid is L-threonine.

It is a further object of the present invention to provide the method described above, wherein said L-amino acid is L-lysine.

It is a further object of the present invention to provide the method described above, wherein said L-amino acid is L-histidine.

It is a further object of the present invention to provide the method described above, wherein said L-amino acid is L-phenylalanine.

It is a further object of the present invention to provide the method described above, wherein said L-amino acid is L-arginine.

It is a further object of the present invention to provide the method described above, wherein said L-amino acid is L-tryptophan.

It is a further object of the present invention to provide the method described above, wherein said L-amino acid is L-glutamic acid.

### BRIEF DESCRIPTION OF THE DRAWINGS

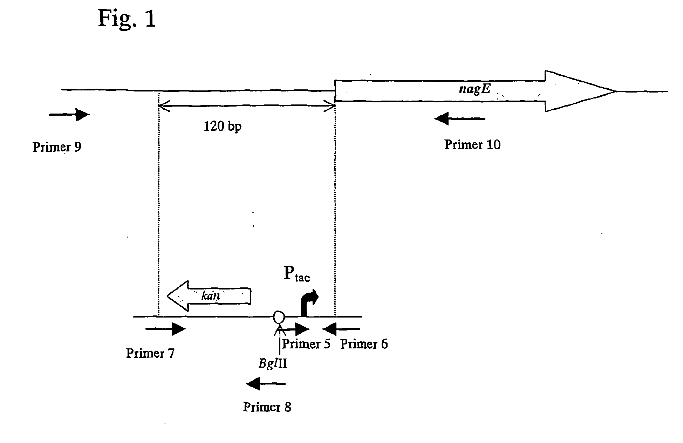

Figure 1 shows the structure of the region upstream of the *nagE* gene in the chromosome of *E. coli* and the structure of an integrated DNA fragment containing the *cat* gene and a P_{tac} promoter.

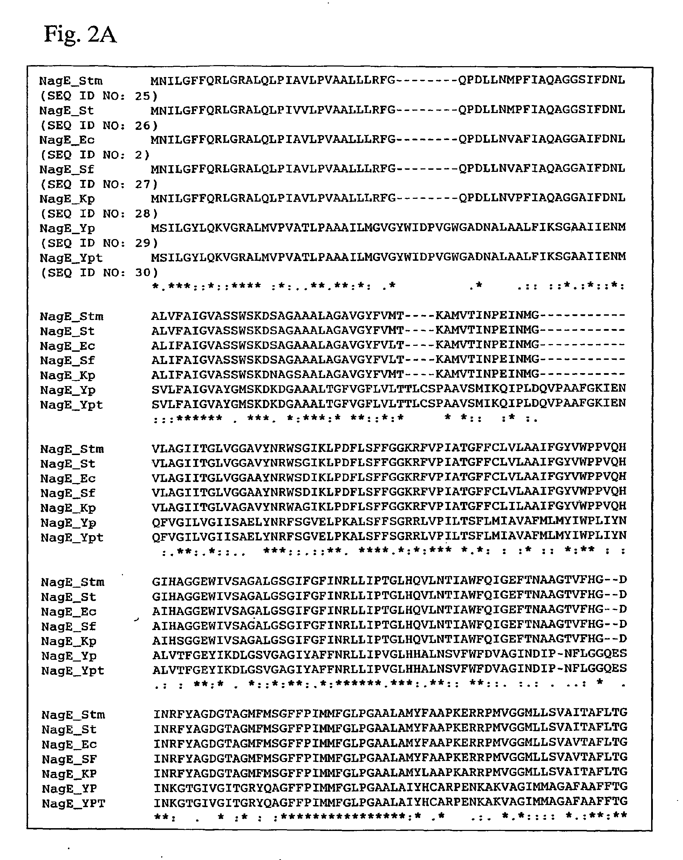

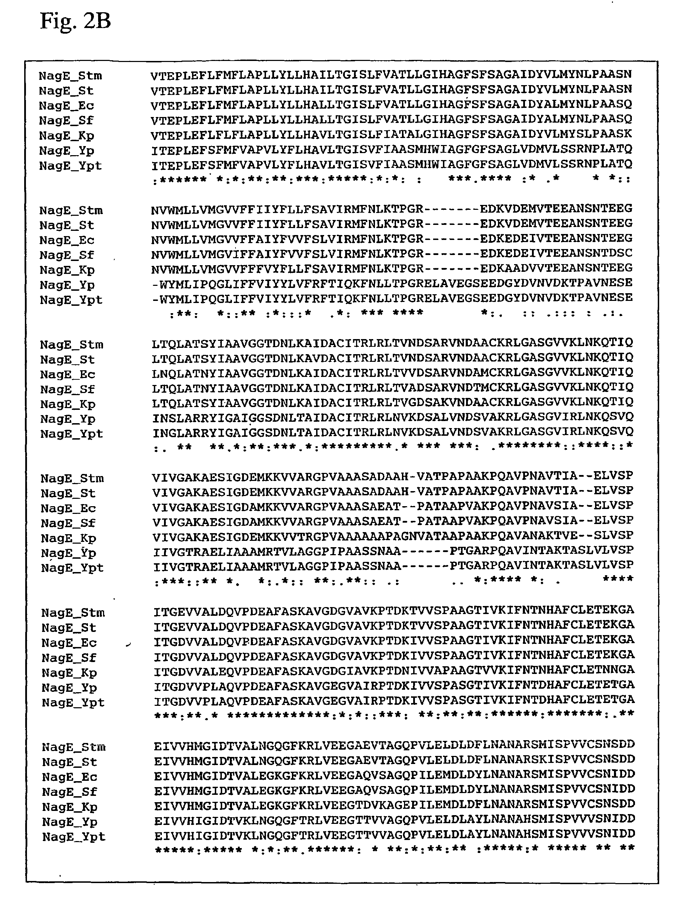

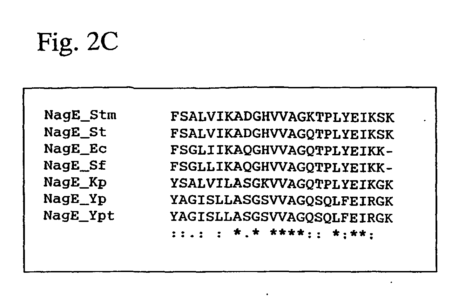

Figure 2 shows the alignment of the primary sequences of N-acetylglucosamine permease from *Salmonella typhimurium* (*Stm*)*, Salmonella typhi* (*St*), *Escherichia coli* (*Ec*), *Shigella flexneri* (*Sf*), *Klebsiella pneumoniae* (*Kp*), *Yersinis pestis* (*Yp*) and *Yersinis pseudotuberculosis* (*Ypt*). The alignment was done by using the PIR Multiple Alignment program (http://pir.georgetown.edu). The identical amino acids are marked by asterisk (*), similar amino acids are marked by colon (:).

### DETAILED DESCRIPTION OF THE PREFERRED EMBODIMENTS

In the present invention, "L-amino acid-producing bacterium" means a bacterium which has an ability to cause accumulation of an L-amino acid in a medium when the bacterium is cultured in the medium. The L-amino acid-producing ability may be imparted or enhanced by breeding. The phrase "L-amino acid-producing bacterium" as used herein also means a bacterium which is able to produce and cause accumulation of an L-amino acid in a culture medium in amount larger than a wild-type or parental strain of *E. coli,* such as *E. coli* K-12, and preferably means that the microorganism is able to cause accumulation in a medium of an amount not less than 0.5 g/L, more preferably not less than 1.0 g/L of the target L-amino acid. "L-amino acids" include L-alanine, L-arginine, L-asparagine, L-aspartic acid, L-cysteine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, and L-valine. L-threonine, L-lysine, L-histidine, L-phenylalanine, L-arginine, L-tryptophan, and L-glutamic acid are particularly preferred.

The *Enterobacteriaceae* family includes bacteria belonging to the genera *Escherichia, Enterobacter, Erwinia, Klebsiella, Pantoea, Providencia, Salmonella, Serratia, Shigella, Morganella,* etc.. Specifically, bacteria classified into the *Enterobacteriaceae* family according to the taxonomy used in the NCBI (National Center for Biotechnology Information) database
(http://www.ncbi.nlm.nih.gov/htbinpost/Taxonomy/wgetorg?mode=Tree&id=1236&1vl=3& keep=1&srchmode=1&unlock) can be used. A bacterium belonging to the genus *Escherichia* or *Pantoea* is preferred.

The phrase "a bacterium belonging to the genus *Escherichia*" means that the bacterium is classified in the genus *Escherichia* according to the classification known to a person skilled in the art of microbiology. Examples of a bacterium belonging to the genus *Escherichia* as used in the present invention include, but are not limited to, *Escherichia coli* (*E. coli*).

The bacterium belonging to the genus *Escherichia* that can be used in the present invention is not particularly limited, however for example, bacteria described by Neidhardt, F.C. et al. (*Escherichia coli* and *Salmonella typhimurium*, American Society for Microbiology, Washington D.C., 1208, Table 1) are encompassed by the present invention.

The phrase "a bacterium belonging to the genus *Pantoea"* means that the bacterium is classified into the genus *Pantoea* according to the classification known to a person skilled in the art of microbiology. Some species of *Enterobacter agglomerans* have been recently re-classified into *Pantoea agglomerans, Pantoea ananatis, Pantoea stewartii,* or the like, based on the nucleotide sequence analysis of 16S rRNA etc. (Int. J. Syst. Bacteriol., 43, 162-173 (1993)).

The bacterium for use in the present invention encompasses a strain of the *Enterobacteriaceae* family which has an ability to produce an L-amino acid and has been modified to enhance an activity of N-acetylglucosamine permease. In addition, the bacterium for use in the present invention encompasses a strain of the *Enterobacteriaceae* family which has an ability to produce an L-amino acid and does not have a native activity of N-acetylglucosamine permease, and has been transformed with a DNA fragment encoding N-acetylglucosamine permease so that the N-acetylglucosamine permease encoded by the DNA fragment is expressed.

The phrase "activity of N-acetylglucosamine permease" means an activity of transporting sugars, such as N-acetylglucosamine and glucose, into the cell. Activity of N-acetylglucosamine permease can be detected and measured by, for example, complementation of growth delay of *crr* mutants as described by Vogler A.P. and Lengeler J.W. (Mol. Gen. Genet., 213(1), 175-8 (1988)).

The phrase "modified to enhance an activity of N-acetylglucosamine permease" means that the activity per cell is higher as compared to that of a non-modified strain, for example, a wild-type strain. Examples of such modifications include increasing the number of N-acetylglucosamine permease molecules per cell, increasing the specific activity per N-acetylglucosamine permease molecule, and so forth. Furthermore, a wild-type strain that may be used for comparison purposes includes, for example, *Escherichia coli* K-12. In the present invention, the amount of accumulated L-amino acid, for example, L-threonine, L-lysine, L-histidine, L-phenylalanine, L-arginine, L-tryptophan, or L-glutamic acid, can be increased in a culture medium as a result of enhancing the intracellular activity of N-acetylglucosamine permease.

Enhancing N-acetylglucosamine permease activity in a bacterial cell can be attained by increasing the expression of the *nagE* gene encoding N-acetylglucosamine permease. Any *nagE* gene derived from bacteria belonging to the genus *Escherichia,* as well as any nag*E* gene derived from other bacteria, such as bacteria belonging to the genus *Bacillus, Klebsiella, Pantoea, Salmonella,* or *Shigella,* may be used as the N-acetylglucosamine permease gene of the present invention. *nagE* genes derived from bacteria belonging to the genus *Escherichia* are preferred.

The phrase "increasing the expression of the gene" means that the expression of the gene is higher than that of a non-modified strain, for example, a wild-type strain. Examples of such modification include increasing the copy number of gene(s) per cell, increasing the expression level of the gene(s), and so forth. The quantity of the copy number of an gene is measured, for example, by restricting the chromosomal DNA followed by Southern blotting using a probe based on the gene sequence, fluorescence *in situ* hybridization (FISH), and the like. The level of gene expression can be measured by various known methods including Northern blotting, quantitative RT-PCR, and the like. Furthermore, wild-type strains that can act as a control includes, for example, *Escherichia coli* K-12 or *Pantoea ananatis* FERM BP-6614 (WO2004099426, AU2004236516A1). As a result of enhancing the intracellular activity of N-acetylglucosamine permease, L-amino acid accumulation, for example L-threonine, L-lysine, L-histidine, L-phenylalanine, L-tryptophan or L-glutamic acid accumulation in a medium is observed.

The *nagE* gene which encodes N-acetylglucosamine permease from *Escherichia coli* has been elucidated (nucleotide numbers complement to numbers 703167 to 705113 in the sequence of GenBank accession NC_000913.2, gi: 16128655). The *nagE* gene is located between *nagB* and *glnS* genes on the chromosome of *E. coli* K-12. Other *nagE* genes which encode N-acetylglucosamine permeases have also been elucidated: *nagE* gene from *Shigella flexneri* (nucleotide numbers complement to numbers 642063 to 644009 in the sequence of GenBank accession NC_004741.1, gi: 30062146); *nagE* gene from *Salmonella enterica* (nucleotide numbers complement to numbers 2257881 to 2259833 in the sequence of GenBank accession NC_004631.1; gi: 29142598), *nagE* from *Yersinia pestis* (nucleotide numbers complement to numbers 1163370 to 1165403 in the sequence of GenBank accession NC_005810.1; gi: 45440916), *nagE* gene from *Lactobacillus plantarum* (nucleotide numbers 2642875 to 2644863 in the sequence of GenBank accession One_004567.1; gi:28379408), and the like. An example of the *nagE* gene from *Escherichia coli* is represented by SEQ ID NO. 1. The amino acid sequence encoded by the *nagE* gene is presented by SEQ ID NO: 2.

Upon being transported into the cell, glucose is phosphorylated by glucokinase, which is encoded by the *glk* gene. So, it is also desirable to modify the bacterium to have enhanced activity of glucokinase. The *glk* gene which encodes glucokinase of *Escherichia coli* has been elucidated (nucleotide numbers 2506481 to 2507446 in the sequence of GenBank accession NC_000913.1, gi:16127994). The *glk* gene is located between the *b2387* and the *b2389* ORFs on the chromosome of *E. coli* K-12.

Under appropriate conditions, the xylose isomerase encoded by the *xylA* gene also efficiently catalyzes the conversion of D-glucose to D-fructose (Wovcha, M.G. et al, Appl Environ Microbiol. 45(4): 1402-4 (1983)). So, it is also desirable to modify the bacterium to have an enhanced activity of xylose isomerase. The *xylA* gene which encodes xylose isomerase of *Escherichia coli* has been elucidated (nucleotide numbers 3728788 to 3727466 in the sequence of GenBank accession NC_000913.2, gi: 49175990). The *xylA* gene is located between *xylB* and *xylF* genes on the chromosome of *E. coli* K-12.

Therefore, *nagE, glk* and *xylA* genes can be obtained by PCR (polymerase chain reaction; refer to White, T.J. et al., Trends Genet., 5, 185 (1989)) utilizing primers prepared based on the known nucleotide sequence of the gene. Genes coding for N-acetylglucosamine permease from other microorganisms can be obtained in a similar manner.

The *nagE* gene derived from *Escherichia coli* is exemplified by a DNA which encodes the following protein (A) or (B):
(A) a protein which has the amino acid sequence shown in SEQ ID NO: 2, or
(B) a variant protein of the amino acid sequence shown in SEQ ID NO: 2, which has an activity of N-acetylglucosamine permease.

The phrase "variant protein" as used in the present invention means a protein which has changes in the sequence, whether they are deletions, insertions, additions, or substitutions of amino acids, but still maintains the desired activity at a useful level, for example, useful for the enhanced production of an L-amino acid. The number of changes in the variant protein depends on the position or the type of amino acid residue in the three dimensional structure of the protein. The number of changes may be 1 to 30, preferably 1 to 15, and more preferably 1 to 5 for the protein (A). These changes in the variants can occur in regions of the protein which are not critical for the function of the protein. This is because some amino acids have high homology to one another so the three dimensional structure or activity is not affected by such a change. These changes in the variant protein can occur in regions of the protein which are not critical for the function of the protein. Therefore, the protein variant (B) may be one which has a homology of not less than 70 %, preferably not less than 80 %, and more preferably not less than 90 %, and most preferably not less than 95 % with respect to the entire amino acid sequence of N-acetylglucosamine permease shown in SEQ ID NO. 2, as long as the activity of N-acetylglucosamine permease is maintained. Homology between two amino acid sequences can be determined using the well-known methods, for example, the computer program BLAST 2.0, which calculates three parameters: score, identity and similarity.

The substitution, deletion, insertion, or addition of one or several amino acid residues should be conservative mutation(s) so that the activity is maintained. The representative conservative mutation is a conservative substitution. Examples of conservative substitutions include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val.

Data comparing the primary sequences of N-acetylglucosamine permease from *Salmonella typhimurium* (*Stm*, SEQ ID NO: 25), *Salmonella typhi* (*St*, SEQ ID NO: 26), *Escherichia coli* (*Ec*, SEQ ID NO: 2), *Shigella flexneri* (*Sf*, SEQ ID NO: 27), *Klebsiella pneumoniae* (*Kp*, SEQ ID NO: 28), *Yersinis pestis* (*Yp*, SEQ ID NO: 29), and *Yersinis pseudotuberculosis* (*Ypt*, SEQ ID NO: 30) show a high level of homology among these proteins (see Figure 2). From this point of view, substitutions or deletions of the amino acid residues which are identical (marked by asterisk) in all the above-mentioned proteins could be crucial for their function. It is possible to substitute similar (marked by colon) amino acids residues by the similar amino acid residues without deterioration of the protein activity. But modifications of other non-conserved amino acid residues may not lead to alteration of the activity of N-acetylglucosamine permease.

The DNA, which encodes substantially the same protein as the N-acetylglucosamine permease described above, may be obtained, for example, by modifying the nucleotide sequence of DNA encoding N-acetylglucosamine permease (SEQ ID NO: 1), for example, by means of the site-directed mutagenesis method so that one or more amino acid residues at a specified site involve deletion, substitution, insertion, or addition. DNA modified as described above may be obtained by conventionally known mutation treatments. Such treatments include hydroxylamine treatment of the DNA encoding proteins of present invention, or treatment of the bacterium containing the DNA with UV irradiation or a reagent such as N-methyl-N'-nitro-N-nitrosoguanidine or nitrous acid.

A DNA encoding substantially the same protein as N-acetylglucosamine permease can be obtained by expressing DNA having a mutation as described above in an appropriate cell, and investigating the activity of any expressed product. A DNA encoding substantially the same protein as N-acetylglucosamine permease can also be obtained by isolating a DNA that is hybridizable with a probe having a nucleotide sequence which contains, for example, the nucleotide sequence shown as SEQ ID NO: 1, under the stringent conditions, and encodes a protein having the N-acetylglucosamine permease activity. The "stringent conditions" referred to herein are conditions under which so-called specific hybrids are formed, and non-specific hybrids are not formed. For example, stringent conditions can be exemplified by conditions under which DNAs having high homology, for example, DNAs having homology of not less than 50%, preferably not less than 60%, more preferably not less than70%, further preferably not less than 80%, and still more preferably not less than 90%, and most preferably not less than 95% are able to hybridize with each other, but DNAs having homology lower than the above are not able to hybridize with each other. Alternatively, stringent conditions may be exemplified by conditions under which DNA is able to hybridize at a salt concentration equivalent to ordinary washing conditions in Southern hybridization, i.e., 1 x SSC, 0.1% SDS, preferably 0.1 x SSC, 0.1% SDS, at 60°C. Duration of washing depends on the type of membrane used for blotting and, as a rule, what is recommended by the manufacturer. For example, recommended duration of washing, for example for the Hybond^{™} N+ nylon membrane (Amersham), under stringent conditions is approximately 15 minutes. Preferably, washing may be performed 2 to 3 times.

A partial sequence of the nucleotide sequence of SEQ ID NO: 1 can also be used as a probe. Probes may be prepared by PCR using primers based on the nucleotide sequence of SEQ ID NO: 1, and a DNA fragment containing the nucleotide sequence of SEQ ID NO: 1 as a template. When a DNA fragment having a length of about 300 bp is used as the probe, the hybridization conditions for washing include, for example, 50°C, 2 x SSC and 0.1% SDS.

The substitution, deletion, insertion, or addition of nucleotides as described above also includes a mutation which naturally occurs (mutant or variant), for example, due to variety in the species or genus of bacterium, and which contains the N-acetylglucosamine permease.

"Transformation of a bacterium with DNA encoding a protein" means introduction of the DNA into a bacterium, for example, by conventional methods. Transformation of this DNA will result in an increase in expression of the gene encoding the protein of present invention, and will enhance the activity of the protein in the bacterial cell. Methods of transformation include any known methods that have hitherto been reported. For example, a method of treating recipient cells with calcium chloride so as to increase permeability of the cells to DNA has been reported for *Escherichia coli* K-12 (Mandel, M. and Higa, A., J. Mol. Biol., 53, 159 (1970)) and may be used.

Methods of gene expression enhancement include increasing the gene copy number. Introducing a gene into a vector that is able to function in a bacterium of the *Enterobacteriaceae* family increases the copy number of the gene. Preferably, low copy vectors are used. Examples of low-copy vectors include but are not limited to pSC101, pMW118, pMW119, and the like. The term "low copy vector" is used for vectors, the copy number of which is up to 5 copies per cell.

Enhancement of gene expression may also be achieved by introduction of multiple copies of the gene into a bacterial chromosome by, for example, a method of homologous recombination, Mu integration, or the like. For example, one act of Mu integration allows introduction of up to 3 copies of the gene into a bacterial chromosome.

Increasing the copy number of the N-acetylglucosamine permease gene can also be achieved by introducing multiple copies of the N-acetylglucosamine permease gene into the chromosomal DNA of the bacterium. In order to introduce multiple copies of the gene into a bacterial chromosome, homologous recombination is carried out using a sequence whose multiple copies exist as targets in the chromosomal DNA. Sequences having multiple copies in the chromosomal DNA include, but are not limited to repetitive DNA, or inverted repeats existing at the end of a transposable element. Also, as disclosed in U.S. Patent No. 5,595,889, it is possible to incorporate the N-acetylglucosamine permease gene into a transposon, and allow it to be transferred to introduce multiple copies of the gene into the chromosomal DNA.

Enhancing gene expression may also be achieved by placing the DNA of the present invention under the control of a potent promoter. For example, the P_{tac} promoter, the *lac* promoter, the *trp* promoter, the *trc* promoter, the P_{R}, or the P_{L} promoters of lambda phage are all known to be potent promoters. The use of a potent promoter can be combined with multiplication of gene copies.

Alternatively, the effect of a promoter can be enhanced by, for example, introducing a mutation into the promoter to increase the transcription level of a gene located downstream of the promoter. Furthermore, it is known that substitution of several nucleotides in the spacer between ribosome binding site (RBS) and the start codon, especially the sequences immediately upstream of the start codon, profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629, 1984). Previously, it was shown that the *rhtA23* mutation is an A-for-G substitution at the-1 position relative to the ATG start codon (ABSTRACTS of 17^{th} International Congress of Biochemistry and Molecular Biology in conjugation with 1997 Annual Meeting of the American Society for Biochemistry and Molecular Biology, San Francisco, California August 24-29, 1997, abstract No. 457). Therefore, it may be suggested that the *rhtA23* mutation enhances the *rhtA* gene expression and, as a consequence, increases the resistance to threonine, homoserine and some other substances transported out of cells.

Moreover, it is also possible to introduce a nucleotide substitution into a promoter region of the N-acetylglucosamine permease gene on the bacterial chromosome, which results in stronger promoter function. The alteration of the expression control sequence can be performed, for example, in the same manner as the gene substitution using a temperature-sensitive plasmid, as disclosed in WO 00/18935 and JP 1-215280 A.

Methods for preparation of plasmid DNA include, but are not limited to digestion and ligation of DNA, transformation, selection of an oligonucleotide as a primer and the like, or other methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

The above-described techniques and guidances for enhancing an activity of N-acetylglucosamine permease are similarly applied to enhancing activities of xylose isomerase and glucokinase.

The bacterium of the present invention can be obtained by introduction of the aforementioned DNAs into bacterium which inherently has the ability to produce L-amino acid. Alternatively, the bacterium of the present invention can be obtained by imparting an ability to produce L-amino acid to the bacterium already containing the DNAs.

### L-threonine producing bacteria

Examples of parent strains for deriving the L-threonine producing bacteria for use in the present invention include, but are not limited to, strains belonging to the genus *Escherichia,* such as *E. coli* TDH-6/pVIC40 (VKPM B-3996) (U.S. Patent No. 5,175,107, U.S. Patent No. 5,705,371), *E. coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E. coli* MG442 (Gusyatiner et al., Genetika (in Russian), 14, 947-956 (1978)), *E. coli* VL643 and VL2055 (EP 1149911 A), and the like.

The strain TDH-6 is deficient in the *thrC* gene, as well as being sucrose-assimilative, and the *ilvA* gene has a leaky mutation. This strain also has a mutation in the *rhtA* gene, which imparts resistance to high concentrations of threonine or homoserine. The strain B-3996 contains the plasmid pVIC40 which was obtained by inserting into RSF1010-derived vector a *thrA*BC* operon which includes a mutant *thrA* gene. This mutant *thrA* gene encodes aspartokinase homoserine dehydrogenase I which has substantially desensitized feedback inhibition by threonine. The strain B-3996 was deposited on November 19, 1987 in the All-Union Scientific Center of Antibiotics (Nagatinskaya Street 3-A, 113105 Moscow, Russian Federation) under the accession number RIA 1867. The strain was also deposited in the Russian National Collection of Industrial Microorganisms (VKPM) (Russia, 117545 Moscow 1, Dorozhny proezd 1) on April 7, 1987 under the accession number B-3996.

Preferably, the bacterium for use in the present invention is additionally modified to enhance expression of one or more of the following genes:
- the mutant *thrA* gene which codes for aspartokinase homoserine dehydrogenase I resistant to feed back inhibition by threonine;
- the *thrB* gene which codes for homoserine kinase;
- the *thrC* gene which codes for threonine synthase;
- the *rhtA* gene which codes for a putative transmembrane protein;
- the *asd* gene which codes for aspartate-β-semialdehyde dehydrogenase; and
- the *aspC* gene which codes for aspartate aminotransferase (aspartate transaminase);

The *thrA* gene which encodes aspartokinase homoserine dehydrogenase I of *Escherichia coli* has been elucidated (nucleotide positions 337 to 2799, GenBank accession NC_000913.2, gi: 49175990). The *thrA* gene is located between *thrL* and *thrB* genes on the chromosome of *E. coli* K-12. The *thrB* gene which encodes homoserine kinase of *Escherichia coli* has been elucidated (nucleotide positions 2801 to 3733, GenBank accession NC_000913.2, gi: 49175990). The *thrB* gene is located between *thrA* and *thrC* genes on the chromosome of *E. coli* K-12. The *thrC* gene which encodes threonine synthase of *Escherichia coli* has been elucidated (nucleotide positions 3734 to 5020, GenBank accession NC_000913.2, gi: 49175990). The *thrC* gene is located between *thrB* gene and *yaaX* opened reading frame on the chromosome of *E. coli* K-12. All three genes are functioning as one threonine operon.

A mutant *thrA* gene which codes for aspartokinase homoserine dehydrogenase I resistant to feed back inhibition by threonine, as well as, the *thrB* and *thrC* genes can be obtained as one operon from well-known plasmid pVIC40 which is presented in the threonine producing *E. coli* strain VKPM B-3996. Plasmid pVIC40 is described in detail in U.S. Patent No. 5,705,371.

The *rhtA* gene exists at 18 min on *E. coli* chromosome close to the *glnHPQ* operon, which encodes components of the glutamine transport system. The *rhtA* gene is identical to ORF1 (*ybiF* gene, numbers 764 to 1651 in the GenBank accession number AAA218541, gi:440181) and located between the *pexB* and *ompX* genes. The unit expressing a protein encoded by the ORF1 has been designated the *rhtA* gene (rht: resistance to homoserine and threonine) gene. Also, it was revealed that the *rhtA23* mutation is an A-for-G substitution at position -1 with respect to the ATG start codon (ABSTRACTS of the 17^{th} International Congress of Biochemistry and Molecular Biology in conjugation with the Annual Meeting of the American Society for Biochemistry and Molecular Biology, San Francisco, California, August 24-29, 1997, abstract No. 457, EP 1013765 A).

The *asd* gene of *E.coli* has already been elucidated (nucleotide positions 3572511 to 3571408, GenBank accession NC_000913.1, gi:16131307), and can be obtained by PCR (polymerase chain reaction; refer to White, T.J. et al., Trends Genet., 5, 185 (1989)), utilizing primers prepared based on the nucleotide sequence of the gene. The *asd* genes of other microorganisms can be obtained in a similar manner.

Also, the *aspC* gene of *E. coli* has already been elucidated (nucleotide positions 983742 to 984932, GenBank accession NC_000913.1, gi:16128895), and can be obtained by PCR. The *aspC* genes of other microorganisms can be obtained in a similar manner Another example of a parent strain for deriving the L-threonine producing bacteria for use in the present invention include the *E. coli* strain MG1655 Δtdh::rhtA* described in Example 1.

More preferably, the bacterium for use in the present invention is further modified so that the *crr* gene which codes for catabolite repression regulator is inactivated in addition to enhancement of expression of the mutant *thrA* gene, the *thrB* gene, the *thrC* gene, the *rhtA* gene, the *asd* gene, and the *aspC* gene.

The *crr* gene which codes for catabolite repression regulator of *E. coli* has been elucidated (nucleotide positions 2533856 to 2534365, GenBank accession NC_000913.2, gi: 49175990). The *crr* gene is located between the *ptsI* and *pdxK* genes on the chromosome of *E. coli* K-12.

The phrase "the *crr* gene is inactivated" means that the target gene is modified so that the modified gene encodes a mutant protein with a decreased activity, or encodes a completely inactive protein. It is also possible that the modified DNA region is unable to provide natural expression of the gene due to the deletion of a part of the gene, the shifting of the reading frame of the gene, the introduction of missense/nonsense mutation, or the modification of an adjacent region of the gene, including sequences controlling gene expression, such as promoter(s), enhancer(s), attenuator(s),_ribosome binding site(s), etc.

Inactivation of the gene can be performed by conventional methods, such as a mutagenesis treatment using UV irradiation or nitrosoguanidine (N-methyl-N'-nitro-N-nitrosoguanidine), a site-directed mutagenesis, a gene disruption using homologous recombination or/and an insertion-deletion mutagenesis (Yu, D. et al., Proc. Natl. Acad. Sci. USA, 2000, 97:12: 5978-83; Datsenko K.A. and Wanner B.L., Proc. Natl. Acad. Sci. USA, 2000, 97:12: 6640-45), also called "Red-driven integration".

### L-lysine producing bacteria

Examples of L-lysine producing bacteria belonging to the genus *Escherichia* include mutants having resistance to an L-lysine analogue. The L-lysine analogue inhibits growth of bacteria belonging to the genus *Escherichia,* but this inhibition is fully or partially desensitized when L-lysine coexists in a medium. Examples of the L-lysine analogue include, but are not limited to, oxalysine, lysine hydroxamate, S-(2-aminoethyl)-L-cysteine (AEC), γ-methyllysine, α-chlorocaprolactam, and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by Lysine is desensitized.

The strain WC196 may be used as an L-lysine producing bacterium of *Escherichia coli.* This bacterial strain was bred by conferring AEC resistance to the strain W3110, which was derived from *Escherichia coli* K-12. The resulting strain was designated as *Escherichia coli* AJ13069, and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and received an accession number of FERM P-14690. Then, it was converted to an international deposit under the provisions of the Budapest Treaty on September 29, 1995, and received an accession number of FERM BP-5252 (U.S. Patent No. 5,827,698).

Examples of parent strains for deriving L-lysine-producing bacteria for use in the present invention also include strains in which expression of one or more genes encoding an L-lysine biosynthetic enzyme are enhanced. Examples of the enzymes involved in L-lysine biosynthesis include, but are not limited to, dihydrodipicolinate synthase (*dapA*), aspartokinase (*lysC*), dihydrodipicolinate reductase (*dapB*), diaminopimelate decarboxylase (*lysA*), diaminopimelate dehydrogenase (*ddh*) (U.S. Patent No. 6,040,160), phosphoenolpyrvate carboxylase (*ppc*), aspartate semialdehyde dehydrogenease (*asd*), nicotinamide adenine dinucleotide transhydrogenase (*pntAB*), and aspartase (*aspA*) (EP 1253195 A).

Examples of parent strains for deriving L-lysine-producing bacteria for use in the present invention also include strains having decreased or eliminated activity of an enzyme that catalyzes a reaction for generating a compound other than L-lysine by branching off from the biosynthetic pathway of L-lysine. Examples of the enzymes that catalyze a reaction for generating a compound other than L-lysine by branching off from the biosynthetic pathway of L-lysine include homoserine dehydrogenase and lysine decarboxylase (U.S. Patent No. 5,827,698).

### L-histidine producing bacteria

Examples of parent strains for deriving L-histidine producing bacteria for use in the present invention include, but are not limited to, strains belonging to the genus *Escherichia,* such as *E. coli* strain 24 (VKPM B-5945, RU2003677); *E. coli* strain 80 (VKPM B-7270, RU2119536); *E. coli* NRRL B-12116 - B12121 (U.S. Patent No. 4,388,405); *E. coli* H-9342 (FERM BP-6675) and H-9343 (FERM BP-6676) (U.S. Patent No. 6,344,347); *E. coli* H-9341 (FERM BP-6674) (EP1085087); *E. coli* AI80/pFM201 (U.S. Patent No. 6258554), and the like.

Examples of parent strains for deriving L-histidine-producing bacteria for use in the present invention also include strains in which expression of one or more genes encoding an L-histidine biosynthetic enzyme are enhanced. Examples of the L-histidine-biosynthetic enzymes include ATP phosphoribosyltransferase (*hisG*), phosphoribosyl AMP cyclohydrolase (*hisI*), phosphoribosyl-ATP pyrophosphohydrolase (*hisIE*), phosphoribosylformimino-5-aminoimidazole carboxamide ribotide isomerase (*hisA*), amidotransferase (*hisH*), histidinol phosphate aminotransferase (*hisC*), histidinol phosphatase (*hisB*), histidinol dehydrogenase (*hisD*), and so forth.

It is known that the genes encoding the L-histidine biosynthetic enzyme (*hisG, hisBHAFI*) are inhibited by L-histidine, and therefore an L-histidine-producing ability can also be efficiently enhanced by introducing a mutation conferring resistance to the feedback inhibition into ATP phosphoribosyltransferase (*hisG*) (Russian Patent Nos. 2003677 and 2119536).

Specific examples of strains having an L-histidine-producing ability include *E. coli* FERM-P 5038 and 5048 which have been introduced with a vector carrying a DNA encoding an L-histidine-biosynthetic enzyme (JP 56-005099 A), *E. coli* strains introduced with *rht,* a gene for an amino acid-export (EP1016710A), *E. coli* 80 strain imparted with sulfaguanidine, DL-1,2,4-triazole-3-alanine, and streptomycin-resistance (VKPM B-7270, Russian Patent No. 2119536), and so forth.

### L-phenylalanine producing bacteria

Examples of parent strains for deriving L-phenylalanine producing strains for use in the present invention include, but are not limited to, strains belonging to the genus *Escherichia,* such as *E. coli* AJ12739 (tyrA::Tn10, tyrR) (VKPM B-8197); *E. coli* HW1089 (ATCC 55371) harboring pheA34 gene (U.S. Patent No. 5,354,672); *E. coli* MWEC101-b (KR8903681); *E. coli* NRRL B-12141, NRRL B-12145, NRRL B-12146 and NRRL B-12147 (U.S. Patent No. 4,407,952), and the like. Also, as a parent strain which can be enhanced in activity of the protein for use in the present invention, L-phenylalanine-producing bacteria belonging to the genus *Escherichia, E. coli* K-12 [W3110 (tyrA)/pPHAB] (FERM BP-3566), *E. coli* K-12 [W3110 (tyrA)/pPHAD] (FERM BP-12659), *E. coli* K-12 [W3110 (tyrA)/pPHATerm] (FERM BP-12662) and *E. coli* K-12 [W3110 (tyrA)/pBR-aroG4, pACMAB] named AJ 12604 (FERM BP-3579) may be used (EP 488424 B1). Furthermore, L-phenylalanine producing bacteria belonging to the genus *Escherichia* with an enhanced activity of a protein encoded by the *yedA* gene or the *yddG* gene may also be used (U.S. Patent Applications 2003/0148473 A1 and 2003/0157667 A1).

### L-arginine producing bacteria

Examples of parent strains for deriving L-arginine producing bacterial for use in the present invention include, but are not limited to, strains belonging to the genus *Escherichia,* such as *E. coli* mutants having resistance to α-methylmethionine, p-fluorophenylalanine, D-arginine, arginine hydroxamate, S-(2-aminoethyl)-cysteine, α-methylserine, β-2-thienylalanine, or sulfaguanidine (JP 56-106598 A); an L-arginine-producing strain into which the *argA* gene encoding N-acetylglutamate synthetase is introduced (EP1170361A1); strains 237 (VKPM B-7925) and 382 (VKPM B-7926) described in EP1170358A1, and the like.

Examples of parent strains for deriving L-arginine producing bacteria for use in the present invention also include strains in which expression of one or more genes encoding an L-arginine biosynthetic enzyme are enhanced. Examples of the L-arginine biosynthetic enzymes include N-acetylglutamyl phosphate reductase (*argC*), ornithine acetyl transferase (*argJ*), N-acetylglutamate kinase (*argB*), acetylornithine transaminase (*argD*), ornithine carbamoyl transferase (*argF*), argininosuccinic acid synthetase (*argG*), argininosuccinic acid lyase (*argH*), and carbamoyl phosphate synthetase. These arginine biosynthetic genes exist on the Arg operon (*argCJBDFRGH*), and are regulated by an arginine repressor encoded by *argR* (J Bacteriol. 2002 Dec;184(23):6602-14). Therefore, disruption of the arginine repressor results in an increase in the expression of the Arg operon, thus enhancing the activities of the L-arginine-producing enzymes (U.S. Patent Application 2002/0045223 A1).

### L-tryptophan producing bacteria

Examples of parent strains for deriving the L-tryptophan-producing bacteria for use in the present invention include, but are not limited to, strains belonging to the genus *Escherichia,* such as *E. coli* JP4735/pMU3028 (DSM10122) and JP6015/pMU91 (DSM10123) deficient in the tryptophanyl-tRNA synthetase encoded by mutant *trpS* gene (U.S. Patent No. 5,756,345); *E. coli* SV164 (pGH5) having the *serA* allele encoding phosphoglycerate dehydrogenase free from feedback inhibition by serine and the *trpE* allele encoding anthranilate synthase free from feedback inhibition by tryptophan (U.S. Patent No. 6,180,373); *E. coli* AGX17 (pGX44) (NRRL B-12263) and AGX6(pGX50)aroP (NRRL B-12264) deficient in the enzyme tryptophanase (U.S. Patent No. 4,371,614); *E. coli* strain AGX17/pGX50,pACKG4-pps in which a phosphoenolpyruvate-producing ability is enhanced (WO9708333, U.S. Patent No. 6,319,696), and the like, may be used.

Previously, it was identified that the *yddG* gene, which encodes a membrane protein which is not involved in a biosynthetic pathway of any L-amino acid, and imparts a microorganism resistance to L-phenylalanine and several amino acid analogues when the wild-type allele of the gene was amplified on a multi-copy vector in the microorganism. Besides, the *yddG* gene can enhance production of L-phenylalanine or L-tryptophan when additional copies are introduced into the cells of the respective producing strain (WO03044192). So, it is desirable that the L-tryptophan-producing bacterium be further modified to have enhanced expression of the *yddG* open reading frame.

Examples of parent strains for deriving the L-tryptophan-producing bacteria for use in the present invention also include strains in which one or more activities of the enzymes selected from anthranilate synthase, phosphoglycerate dehydrogenase, and tryptophan synthase are enhanced. The anthranilate synthase and phosphoglycerate dehydrogenase are both subject to feedback inhibition by L-tryptophan and L-serine, so that a mutation desensitizing the feedback inhibition may be introduced into these enzymes. Specific examples of strains having such a mutation include a *E. coli* SV164 which harbors desensitized anthranilate synthase and a transformant strain obtained by introducing into the *E. coli* SV164 the plasmid pGH5 (WO 94/08031), which contains a mutant *serA* gene encoding feedback-desensitized phosphoglycerate dehydrogenase.

Examples of parent strains for deriving the L-tryptophan-producing bacteria for use in the present invention also include strains into which the tryptophan operon which contains a gene encoding desensitized anthranilate synthase has been introduced (JP 57-71397 A, JP 62-244382 A, U.S. Patent No. 4,371,614). Moreover, L-tryptophan-producing ability may be imparted by enhancing expression of a gene which encodes tryptophan synthase, among tryptophan operon (*trpBA*). The tryptophan synthase consists of a and β subunits which are encoded by *trpA* and *trpB,* respectively.

### L-glutamic acid producing bacteria

Examples of parent strains for deriving L-glutamic acid-producing bacteria for use in the present invention include, but are not limited to, strains such as *E. coli* VL334thrC⁺ (EP 1172433). *E. coli* VL334 (VKPM B-1641) is an L-isoleucine and L-threonine auxotrophic strain having mutations in *thrC* and *ilvA* genes (U.S. Patent No. 4,278,765). A wild-type allele of the *thrC* gene was transferred by the method of general transduction using a bacteriophage P1 grown on wild-type *E. coli* K12 (VKPM B-7) cells. As a result, an L-isoleucine auxotrophic strain VL334thrC⁺ (VKPM B-8961) was obtained. This strain is able to produce L-glutamic acid.

Examples of parent strains for deriving the L-glutamic acid-producing bacteria for use in the present invention include, but are not limited to, strains in which expression of one or more genes encoding an L-glutamic acid biosynthetic enzyme are enhanced. Examples of the enzymes involved in L-glutamic acid biosynthesis include glutamate dehydrogenase, glutamine synthetase, glutamate synthetase, isocitrate dehydrogenase, aconitate hydratase, citrate synthase, phosphoenolpyruvate carboxylase, pyruvate carboxylase, pyruvate dehydrogenase, pyruvate kinase, phosphoenolpyruvate synthase, enolase, phosphoglyceromutase, phosphoglycerate kinase, glyceraldehyde-3-phophate dehydrogenase, triose phosphate isomerase, fructose bisphosphate aldolase, phosphofructokinase, and glucose phosphate isomerase.

Examples of strains modified so that expression of the citrate synthetase gene, the phosphoenolpyruvate carboxylase gene, and/or the glutamate dehydrogenase gene is/are enhanced include those disclosed in EP1078989A, EP955368A, and EP952221A.

Examples of parent strains for deriving the L-glutamic acid-producing bacteria for use in the present invention also include strains having decreased or eliminated activity of an enzyme that catalyzes synthesis of a compound other than L-glutamic acid, and branching off from an L-glutamic acid biosynthesis pathway. Examples of such enzymes include isocitrate lyase, α-ketoglutarate dehydrogenase, phosphotransacetylase, acetate kinase, acetohydroxy acid synthase, acetolactate synthase, formate acetyltransferase, lactate dehydrogenase, and glutamate decarboxylase. Bacteria belonging to the genus *Escherichia* deficient in the a-ketoglutarate dehydrogenase activity or have a reduced α-ketoglutarate dehydrogenase activity, and methods for obtaining them are described in U.S. Patent Nos. 5,378,616 and 5,573,945. Specifically, these strains include the following:
*E. coli* W3110sucA::Kmr
*E. coli* AJ12624 (FERM BP-3853)
*E. coli* AJ12628 (FERM BP-3854)
*E. coli* AJ12949 (FERM BP-4881)
*E. coli* W3110sucA::Kmr is a strain which is obtained by disrupting the α-ketoglutarate dehydrogenase gene (hereinafter referred to as "sucA gene") of *E. coli* W3110. This strain is completely deficient in the α-ketoglutarate dehydrogenase.

Other examples of L-glutamic acid-producing bacterium include those which belong to the genus *Escherichia* and have resistance to an aspartic acid antimetabolite. These strains can also be deficient in the α-ketoglutarate dehydrogenase activity and include, for example, *E. coli* AJ13199 (FERM BP-5807) (U.S. Patent No. 5.908,768), FFRM P-12379, which additionally has a low L-glutamic acid decomposing ability (U.S. Patent No. 5,393,671); AJ13138 (FERM BP-5565) (U.S. Patent No. 6,110,714), and the like.

Examples of L-glutamic acid-producing bacteria, include mutant strains belonging to the genus *Pantoea* which are deficient in the α-ketoglutarate dehydrogenase activity or have a decreased α-ketoglutarate dehydrogenase activity, and can be obtained as described above. Such strains include *Pantoea ananatis* AJ13356 (U.S. Patent No. 6,331,419). *Pantoea ananatis* AJ13356 was deposited at the National Institute of Bioscience and Human-Technology, Agency of Industrial Science and Technology, Ministry of International Trade and Industry (currently, National Institute of Advanced Industrial Science and Technology, International Patent Organism Depositary, Central 6, 1-1, Higashi 1-Chome, Tsukuba-shi, Ibaraki-ken, 305-8566, Japan) on February 19, 1998 and received an accession number of FERM P-16645. It was then converted to an international deposit under the provisions of the Budapest Treaty on January 11,1999 and received an accession number of FERM BP-6615. *Pantoea ananatis* AJ13356 is deficient in α-ketoglutarate dehydrogenase activity as a result of disruption of the aKGDH-E1 subunit gene (sucA). The above strain was identified as *Enterobacter agglomerans* when it was isolated and deposited as the *Enterobacter agglomerans* AJ13356. However, it was recently re-classified as *Pantoea ananatis* on the basis of nucleotide sequencing of 16S rRNA and so forth. Although AJ13356 was deposited at the aforementioned depository as *Enterobacter agglomerans,* for the purposes of this specification, they are described as *Pantoea ananatis.*

### Production of L-amino acids

Oxaloacetate (OAA) serves as a substrate for the reaction which results in synthesis of Thr and Lys. OAA results from a reaction of PEP with phosphoenol pyrvate carboxlase (PEPC) functioning as a catalyst. Therefore, elevation of the PEPC concentration in a cell can be very important for fermentative production of these amino acids. When using glucose as the carbon source in fermentation, glucose is internalized by the glucose-phosphontransferase (Glc-PTS) system. This system consumes PEP, and proteins in the PTS are encoded by ptsG and ptsHIcrr. During internalization, one molecule of PEP and one molecule of pyruvate (Pyr) are generated from one molecule of glucose.

An L-threonine-producing strain and an L-lysine-producing strain which have been modified to have an ability to utilize sucrose (Scr-PTS) have higher productivity of these amino acids when cultured in sucrose rather than glucose (EP 1149911 A2). It is believed that three molecules of PEP and one molecule of Pyr are generated from one molecule of sucrose by the Scr-PTS, increasing the ratio of PEP/Pyr, and thereby facilitating the synthesis of Thr and Lys from sucrose. Furthermore, it has been reported that Glc-PTS is subject to several expression controls (Postma P.W. et al., Microbiol Rev., 57(3), 543-94 (1993); Clark B. et al. J. Gen. Microbiol., 96(2), 191-201 (1976); Plumbridge J., Curr. Opin. Microbiol., 5(2), 187-93 (2002); Ryu S. et al., J. Biol. Chem., 270(6):2489-96 (1995)), and hence it is possible that the incorporation of glucose itself can be a rate-limiting step in amino acid fermentation.

Increasing the ratio of PEP/Pyr even more by increasing expression of the *nagE* gene in a threonine-producing strain, a lysine-producing strain, a histidine-producing strain, a phenylalanine-producing strain, an arginine-producing strain, a tryptophan-producing strain and/or a glutamic acid-producing strain should further increase amino acid production. Because four molecules of PEP are generated from two molecules of glucose, the ratio of PEP/Pyr is expected to be greatly improved. Due to the increased expression of the *nagE* gene, removal of the expression control glc-PTS is expected.

The method for producing an L-amino acid of the present invention includes the steps of cultivating the bacterium for use in the present invention in a culture medium, allowing L-amino acid to accumulate in the culture medium, and collecting L-amino acid from the culture medium. Furthermore, the method of present invention includes a method for producing L-threonine, L-lysine, L-histidine, L-phenylalanine, L-arginine, L-tryptophan, or L-glutamic acid, including the steps of cultivating the bacterium for use in the present invention in a culture medium, allowing L-threonine, L-lysine, L-histidine, L-phenylalanine, L-arginine, L-tryptophan, or L-glutamic acid to accumulate in the culture medium, and collecting L-threonine, L-lysine, Histidine, L-phenylalanine, L-arginine, L-tryptophan, or L-glutamic acid from the culture medium.

In the present invention, the cultivation, collection, and purification of L-amino acids from the medium and the like may be performed by conventional fermentation methods wherein an L-amino acid is produced using a bacterium.

The culture medium may be either synthetic or natural, so long as the medium includes a carbon source, a nitrogen source, minerals, and if necessary, appropriate amounts of nutrients which the bacterium requires for growth. The carbon source may include various carbohydrates such as glucose and sucrose, and various organic acids. Depending on the mode of assimilation of the bacterium used, alcohols, including ethanol and glycerol may be used. As the nitrogen source, various ammonium salts such as ammonia and ammonium sulfate, other nitrogen compounds such as amines, a natural nitrogen source such as peptone, soybean-hydrolysate, and digested fermentative microorganisms may be used. As minerals, potassium monophosphate, magnesium sulfate, sodium chloride, ferrous sulfate, manganese sulfate, calcium chloride, and the like may be used. As vitamins, thiamine, yeast extract, and the like may be used. Additional nutrients may be added to the medium, if necessary. For example, if the bacterium requires an L-amino acid for growth (L-amino acid auxotrophy), a sufficient amount of the L-amino acid may be added to the cultivation medium.

The cultivation is preferably performed under aerobic conditions such as a shaking culture, and stirring culture with aeration, at a temperature of 20 to 40°C, preferably 30 to 38°C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to accumulation of the target L-amino acids in the liquid medium.

After cultivation, solids such as cells can be removed from the liquid medium by centrifugation or membrane filtration, and then target L-amino acids can be collected and purified by ion-exchange, concentration, and crystallization methods.

### Examples

The present invention will be more concretely explained below with reference to the following non-limiting examples.

### Example 1. Preparation of the E. coli strain MG1655 Δtdh::rhtA*.

The L-threonine producing *E. coli* strain MG1655 Δtdh, rhtA* (pVIC40) was constructed by inactivation of the native *tdh* gene in *E. coli* MG1655 (ATCC700926) using the *cat* gene followed by introduction of an *rlatA23* mutation which confers resistance to high concentrations of threonine (>40 mg/ml) and homoserine (>5 mg/ml). Then, the resulting strain was transformed with plasmid pVIC40 from *E. coli* VKPM B-3996. Plasmid pVIC40 is described in detail in the US patent 5,705, 371.

To substitute the native *tdh* gene, a DNA fragment carrying the chloramphenicol resistance marker (Cm^{R}) encoded by the *cat* gene was integrated into the chromosome of *E. coli* MG1655 in place of the native gene by the method described by Datsenko K.A. and Wanner B.L. (Proc.Natl.Acad.Sci.USA, 2000, 97, 6640-6645) which is also called "Red-mediated integration " and/or "Red-driven integration". The recombinant plasmid pKD46 (Datsenko, K.A., Wanner, B.L., Proc.Natl.Acad.Sci.USA, 2000, 97, 6640-6645) with the thermosensitive replicon was used as the donor of the phage λ-derive genes responsible for the Red-mediated recombination system. *E. coli* BW25113 containing the recombinant plasmid pKD46 can be obtained from the *E. coli* Genetic Stock Center, Yale University, New Haven, USA, the accession number of which is CGSC7630. The recombinant plasmid pKD46 (Datsenko, K.A., Wanner, B.L., Proc.Natl.Acad.Sci.USA, 2000, 97, 6640-6645) with the thermosensitive replicon was used as the donor of the phage λ-derive genes responsible for the Red-mediated recombination system. *E. coli* BW25113 containing the recombinant plasmid pKD46 can be obtained from the *E. coli* Genetic Stock Center, Yale University, New Haven, USA, the accession number of which is CGSC7630.

A DNA fragment containing a Cm^{R} marker encoded by the cat gene was obtained by PCR using the commercially available plasmid pACYC184 (GenBank/EMBL accession number X06403, "Fermentas", Lithuania) as the template, and primers P1 (SEQ ID NO: 3) and P2 (SEQ ID NO: 4). Primer P1 contains 35 nucleotides homologous to the 5'-region of the *tdh* gene introduced into the primer for further integration into the bacterial chromosome. Primer P2 contains 32 nucleotides homologous to the 3'-region of the *tdh* gene introduced into the primer for further integration into the bacterial chromosome.

PCR was provided using the "Gene Amp PCR System 2700" amplificatory (Applied Biosystems). The reaction mixture (total volume - 50 µl) consisted of 5 µl of 10x PCR-buffer with 25 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added in the reaction mixture as a template DNA for the PCR amplification. The temperature profile was the following: initial DNA denaturation for 5 min at 95 °C, followed by 25 cycles of denaturation at 95 °C for 30 sec, annealing at 55 °C for 30 sec, elongation at 72 °C for 40 sec; and the final elongation for 5 min at 72 °C. Then, the amplified DNA fragment was purified by agarose gel-electrophoresis, extracted using "GenElute Spin Columns" (Sigma, USA), and precipitated by ethanol.

The obtained DNA fragment was used for electroporation and Red-mediated integration into the bacterial chromosome of the *E. coli* MG1655/pKD46.

MG1655/pKD46 cells were grown overnight at 30 °C in the liquid LB-medium with addition of ampicillin (100 µg/ml), then diluted 1:100 by the SOB-medium (Yeast extract, 5 g/l; NaCl, 0.5 g/l; Tryptone, 20 g/l; KCl, 2.5 mM; MgCl₂, 10 mM) with addition of ampicillin (100 µg/ml) and L-arabinose (10 mM) (arabinose is used for inducing the plasmid containing the genes of the Red system) and grown at 30 °C to reach the optical density of the bacterial culture OD₆₀₀=0.4-0.7. The grown cells from 10 ml of the bacterial culture were washed 3 times with ice-cold de-ionized water, followed by suspending in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions. Shocked cells were added to 1-ml of SOC medium (Sambrook et al, "Molecular Cloning A Laboratory Manual, Second Edition", Cold Spring Harbor Laboratory Press (1989)), incubated 2 hours at 37°C, and then were spread onto L-agar containing 25 µg/ml of chloramphenicol. Colonies grown for 24 hours were tested for the presence of Cm^{R} marker instead of the native *tdh* gene by PCR using primers P3 (SEQ ID NO: 5) and P4 (SEQ ID NO: 6). For this purpose, a freshly isolated colony was suspended in 20µl water and then 1µl of obtained suspension was used for PCR. The temperature profile follows: initial DNA denaturation for 5 min at 95 °C; then 30 cycles of denaturation at 95 °C for 30 sec, annealing at 55 °C for 30 sec and elongation at 72 °C for 30 sec; the final elongation for 5 min at 72 °C. A few Cm^{R} colonies tested contained the desired 1104 bp DNA fragment, confirming the presence of Cm^{R} marker DNA instead of 1242 bp fragment of *tdh* gene. One of the obtained strains was cured from the thermosensitive plasmid pKD46 by culturing at 37 °C and the resulting strain was named as *E. coli* MG1655Δtdh.

Then, the *rhtA23* mutation from the strain VL614rhtA23 (Livshits V.A. et al, 2003, Res.Microbiol., 154:123-135) was introduced into obtained strain MG1655 Δtdh resulting in the strain MG1655 Δtdh, rhtA*. The rhtA23 is a mutation which confers resistance to highconcentrations of threonine (>40 mg/ml) and homoserine (>5 mg/ml). For that purpose the strain MG1655 Δtdh was infected with phage P1ᵥᵢᵣ grown on the donor strain VL614rhtA23. The transductants were selected on M9 minimal medium containing 8 mg/ml homoserine and 0.4% glucose as the sole carbon source.

### Example 2. Substitution of the native promoter region of the nagE gene in E. coli by P_{tac} promoter.

To substitute the native promoter region of the *nagE* gene, a DNA fragment carrying a modified P_{tac} promoter (with deletion of one nucleotide in LacI-binding site region that led to absence of LacI-dependent repression) and kanamycin resistance marker (Km^{R}) encoded by the *kan* gene was integrated into the chromosome of *E. coli* MG1655 Δtdh::rhtA in place of the native promoter region by the method described by Datsenko K.A. and Wanner B.L. (Proc.Natl.Acad.Sci.USA, 2000, 97, 6640-6645) which is also called "Red-mediated integration " and/or "Red-driven integration".

The modified P_{tac} promoter was obtained by PCR using the commercially available plasmid pKK223-3 (Pharmacia) as a template and primers P5 (SEQ ID NO: 7) and P6 (SEQ ID NO: 8). Primer P5 contains a *Bgl*II recognition site at the 3'-end thereof, which is necessary for further joining to the *kan* gene and primer P6 contains 29 nucleotides homologous to the 3'-region of the P_{tac} promoter (with deletion of C- nucleotide in LacI-binding site region).

A DNA fragment containing a Km^{R} marker encoded by the *kan* gene was obtained by PCR using the commercially available plasmid pACYC177 (GenBank/EMBL accession number X06402, "Fermentas", Lithuania) as the template, and primers P7 (SEQ ID NO: 9) and P8 (SEQ ID NO: 10). Primer P7 contains 41 nucleotides homologous to the region located 120 bp upstream of the start codon of the *nagE* gene introduced into the primer for further integration into the bacterial chromosome and primer P8 contains a *Bgl*II recognition site at the 5'-end thereof, which is necessary for further joining to the modified P_{tac} promoter.

PCR was provided using the "Gene Amp PCR System 2700" amplificatory (Applied Biosystems). The reaction mixture (total volume - 50 µl) consisted of 5 µl of 10x PCR-buffer with 25 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added to the reaction mixture as a template DNA for the PCR amplification. The temperature profile was the following: initial DNA denaturation at 95 °C for 5 min, followed by 25 cycles of denaturation at 95 °C for 30 sec, annealing at 55°C for 30 sec, elongation at 72 °C for 20 sec for P_{tac} promoter and 50 sec for *kan* gene; and the final elongation for 5 min at 72°C. Then, the amplified DNA fragment was purified by agarose gel-electrophoresis, extracted using "GenElute Spin Columns" (Sigma, USA), and precipitated by ethanol.

Each of the two above-described DNA fragments was treated with *Bgl*II restrictase and ligated. The ligation product was amplified by PCR using primers P6 (SEQ ID NO: 8) and P7 (SEQ ID NO: 9) The amplified *kan*-P*_{tac}nagE* DNA fragment was purified by agarose gel-electrophoresis, extracted using "GenElute Spin Columns" (Sigma, USA) and precipitated by ethanol. The obtained DNA fragment was used for electroporation and Red-mediated integration into the bacterial chromosome of the *E. coli* MG1655Δtdh::rhtA/pKD46.

MG1655 Δtdh, rhtA/pKD46 cells were grown overnight at 30 °C in the liquid LB-medium with the addition of ampicillin (100 µg/ml), then diluted 1:100 with the SOB-medium (Yeast extract, 5 g/l; NaCl, 0.5 g/l; Tryptone, 20 g/l; KCl, 2.5 mM; MgCl₂,10 mM) with the addition of ampicillin (100 µg/ml) and L-arabinose (10 mM) (arabinose is used for inducing the plasmid encoding genes of the Red system) and grown at 30°C to reach the optical density of the bacterial culture OD₆₀₀=0.4-0.7. Grown cells from 10 ml of the bacterial culture were washed 3 times with ice-cold de-ionized water, followed by suspending in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions.

Shocked cells were added to 1-ml of SOC medium (Sambrook et al, "Molecular Cloning A Laboratory Manual, Second Edition", Cold Spring Harbor Laboratory Press (1989)), incubated 2 hours at 37 °C, and then were spread onto L-agar containing 20 µg/ml of kanamycin.

Colonies grown within 24 hours were tested for the presence of Km^{R} marker, instead of the native promoter region of the *nagE* gene by PCR using primers P9 (SEQ ID NO: 11) and P10 (SEQ ID NO: 12). For this purpose, a freshly isolated colony was suspended in 20µl water and then 1µl of obtained suspension was used for PCR. The following temperature profile was used: initial DNA denaturation for 10 min at 95 °C; then 30 cycles of denaturation at 95 °C for 30 sec, annealing at 55°C for 30 sec and elongation at 72 °C for 1.5 min; the final elongation for 5 min at 72 °C. A few Km^{R} colonies tested contained the desired 1700 bp DNA fragment, confirming the presence of Km^{R} marker DNA instead of 600 bp native promoter region of *nagE* gene (see Figure 1). One of the obtained strains was cured from the thermosensitive plasmid pKD46 by culturing at 37°C and the resulting strain was named as *E. coli* MG1655Δtdh,rhtA*, P_{tac}nagE.

### Example 3. Effect of increasing the nagE gene expression on L-threonine production.

To evaluate the effect of enhancing expression of the *nagE* gene on threonine production, both *E. coli* strains MG1655Δtdh, rhtA*, P_{tac}nagE and MG1655Δtdh, rhtA* were transformed with plasmid pVIC40.

Then *E. coli* strains MG1655Δtdh, rhtA*, P_{tac}nagE and MG1655Δtdh, rhtA* were each cultivated at 37 ºC for 18 hours in a nutrient broth, and 0.3 ml of each of the obtained cultures was inoculated into 3 ml of fermentation medium having the following composition in a 20x200 mm test tube and cultivated at 37 ºC for 24 hours with a rotary shaker.

After cultivation, the accumulated amount of L-threonine in the medium can be determined by paper chromatography using the following mobile phase: butanol : acetic acid : water =4:1:1 1(v/v). A solution (2%) of ninhydrin in acetone can be used as a visualizing reagent. A spot containing L-threonine can be cut off, L-threonine can be eluted in 0.5 % water solution of CdCl₂, and the amount of L-threonine can be estimated spectrophotometrically at 540 nm. The results of test tube fermentation are shown in Table 1.

The composition of the fermentation medium (g/l) is as follows:

| | |
|---|---|
| Glucose | 40.0 |
| (NH₄)₂SO₄ | 16.0 |
| K₂HPO₄ | 0.7 |
| MgSO₄·7H₂O | 1.0 |
| MnSO₄·5H₂O | 0.01 |
| FeSO₄·7H₂O | 0.01 |
| Thiamine hydrochloride | 0.002 |
| Yeast extract | 2.0 |
| L-isolucine | 0.01 |
| CaCO₃ | 33.0 |

MgSO₄·7H₂O and CaCO₃ were each sterilized separately.

**Table 1**

| Strain | OD₅₄₀ | Thr, g/l |
|---|---|---|
| MG1655Δtdh, rhtA* (pVIC40) ¹ Km^{R} | 30.5±0.4 | 6.0±0.3 |
| MG1655Δtdh, rhtA*, P_{tac}nagE(pVIC40) ² Cm^{R}, Km^{R} | 26.8±0.7 | 7.3±0.5 |
| MG1655Δtdh, rhtA*, P_{tac}nagE, Δcrr (pVIC40) ² Cm^{R}, Km^{R}, Tet^{R} | 26.5±0.6 | 8.1±0.3 |

| | | |
|---|---|---|
| Notes: ¹ - data of 5 test-tubes fermentation , ²- data of 12 test-tubes fermentation. | | |

It can be seen from the Table 1, MG1655Δtdh, rhtA*, P_{tac}nagE accumulated a higher amount of L-threonine as compared with MG1655Δtdh, rhtA*, in which the expression amount of N-acetylglucosamine permease is not increased.

### Example 4. Construction of E. coli MG1655Δtdh, rhtA*, P_{tac}nagE, Δcrr and effect of additional inactivation of the crr gene on L-threonine production

Strain MG1655Δtdh, rhtA*, P_{tac}nagE, Δcrr was constructed by inactivation of the native *crr* gene in *E. coli* MG1655Δtdh, rhtA*, P_{tac}nagE using the *tetAtetR* genes.

To substitute the native *crr* gene, a DNA fragment carrying tetracycline resistance marker (Tet^{R}) encoded by the *tetAtetR* genes was integrated into the chromosome of *E. coli* MG1655Δtdh, rhtA*, P_{tac}nagE by the method described by Datsenko K.A. and Wanner B.L. (Proc.Natl.Acad.Sci.USA, 2000, 97, 6640-6645).

A DNA fragment containing a tet^{R} marker encoded by the *tetAtetR* genes was obtained by PCR using the genomic DNA of *E.coli* Tn10 (VKPM B-5993) as the template, and primers P11 (SEQ ID NO: 13) and P12 (SEQ ID NO: 14). Each of primers contains 40 nucleotides homologous to the 5'-region and 3'-region of the *crr* gene accordingly which were introduced into the primers for further integration into the bacterial chromosome. This strain VKPM B-5993 is available from the Russian National Collection of Industrial Microorganisms (Russia, 113545 Moscow, 1 Dorozhny proezd, 1) upon request.

PCR was provided using the "Gene Amp PCR System 2700" amplificatory (Applied Biosystems). The reaction mixture (total volume - 50 µl) consisted of 5 µl of 10x PCR-buffer with 25 mM MgCl₂ ("Fermentas", Lithuania), 200 µM each of dNTP, 25 pmol each of the exploited primers and 1 U of Taq-polymerase ("Fermentas", Lithuania). Approximately 20 ng of the genomic DNA was added to the reaction mixture as a template DNA for the PCR amplification. The temperature profile was the following: initial DNA denaturation for 5 min at 95°C, followed by 35 cycles of denaturation at 95 °C for 30 sec, annealing at 54 °C for 30 sec, elongation at 72 °C for 1 min 30 sec; and the final elongation for 5 min at 72 °C. Then, the amplified DNA fragment was purified by agarose gel-electrophoresis, extracted using "GenElute Spin Columns" ("Sigma", USA) and precipitated by ethanol.

The obtained DNA fragment was used for electroporation and Red-mediated integration into the bacterial chromosome of the *E. coli* MG1655/pKD46.

The conditions of the *tetAtetR* fragment electroporation into *E. coli* MG1655Δtdh,rhtA, P_{tac}nagE strain was the same as described above.

Shocked cells were added to 1ml of SOC medium (Sambrook et al, "Molecular Cloning A Laboratory Manual, Second Edition", Cold Spring Harbor Laboratory Press (1989)), incubated at 37°C for 2 hours, and then were spread onto L-agar containing 50 µg/ml of tetracycline.

Colonies grown within 24 hours were tested for the presence of Tet^{R} marker instead of the native *crr* gene by PCR using primers P13 (SEQ ID NO: 15) and P14 (SEQ ID NO: For this purpose, a freshly isolated colony was suspended in 20µl water and then 1µl of obtained suspension was used for PCR. The temperature profile follows: initial DNA denaturation for 10 min at 95 °C; then 30 cycles of denaturation at 95 °C for 30 sec, annealing at 54 °C for 30 sec and elongation at 72°C for 1,5 min; the final elongation for 5 min at 72°C. A few Tet^{R} colonies tested contained the desired 2040 bp DNA fragment, confirming the presence of Tet^{R} marker DNA instead of the native *crr* gene of 670 bp. One of the obtained strains was cured of the thermosensitive plasmid pKD46 by culturing at 37 °C and the resulting strain was named as *E. coli* MG1655Δtdh,rhtA,P_{tac}nagE, Δcrr.

*E. coli* MG1655Δtdh, rhtA*, P_{tac}nagE, Δcrr was cultivated at 37 ºC for 18 hours in a nutrient broth and 0.3 ml of each of the obtained cultures was inoculated into 3 ml of fermentation medium in a 20x200 mm test tube and cultivated at 37ºC for 24 hours with a rotary shaker as described above. The results of test tube fermentation are shown in Table 1.

### Example 5. Effect of increasing the nagE gene expression on L-lysine production.

To test the effect of enhanced expression of the *nagE* gene under the control of P*_{lac}* promoter on L-lysine production, a DNA fragment comprising the *nagE* gene was cloned into pMW219 (Takara Shuzo, Japan). Specific procedures of constructing a plasmid used for enhancing expression of the *nagE* gene were as follows; DNA fragments comprising the *nagE* gene were amplified by PCR method, using primers P15 (SEQ ID NO: 17) and P16 (SEQ ID NO: 18), which included the *Hind*III and *Xba*I sites, respectively. PCR was provided using the "Pyrobest DNA Polymerase" (Applied Takara Shuzo, Japan). Approximately 20 ng of the genomic DNA of *E. coli* K-12 MG1655 was used as a template DNA for the PCR amplification. The temperature profile was the following: initial DNA denaturation for 1 min at 94 °C, followed by 25 cycles of denaturation at 98 °C for 10 sec, annealing at 55 °C for 30 sec, elongation at 72 °C for 2 min; and the final elongation for 5 min at 72 °C. Then, the amplified DNA fragment was purified by agarose gel-electrophoresis, extracted using "GenElute Spin Columns" ("Sigma", USA) and precipitated by ethanol. The PCR product and the vector pMW219 were digested by *Hind*III and *Xba*I, and then ligated each other by 2 x Ligation Mit (Nippon Gene, Japan). Thus the plasmid pM-*nagE* containing the *nagE* gene under the control of the P*_{lac}* promoter was constructed.

The lysine-producing *E. coli* strain WC196 (pCABD2) was transformed with pMW219 for a control and pM-*nagE*, and WC196 (pCABD2, pMW219) and WC196 (pCABD2, pM*-nagE*) were constructed, respectively. pCABD2 is a plasmid comprising a *dapA* gene coding for a dihydrodipicolinate synthase having a mutation which desensitizes feedback inhibition by L-lysine, a *lysC* gene coding for aspartokinase III having a mutation which desensitizes feedback inhibition by L-lysine, a *dapB* gene coding for a dihydrodipicolinate reductase gene, and a *ddh* gene coding for diaminopimelate dehydrogenase (U.S. Patent No. 6,040,160).

Both *E. coli* strains WC196 (pCABD2, pMW219) and WC196 (pCABD2, pM-nagE) were cultured in the L-medium containing 25 mg/l of kanamycin and 20 mg/l of streptomycin at 37°C until OD₆₀₀ became about 0.6. Then, an equal volume of 40% glycerol solution was added to each culture broth, stirred, and then divided into appropriate volumes, and stored at -80°C. These are referred to herein as glycerol stocks.

The glycerol stocks of these strains were thawed, and 100 µl of each stock was uniformly plated on an L-plate containing 25 mg/l of kanamycin and 20 mg/l of streptomycin and incubated at 37°C for 24 hours. About 1/8 of the cells collected from the plate were inoculated into 20ml of the fermentation medium containing 25 mg/l of kanamycin and 20 mg/l of streptomycin in a 500 ml Sakaguchi flask, and cultured at 37°C for 24 hours on a culturing apparatus with shaking by reciprocal movement. After cultivation, the amount of lysine which had accumulated in the medium was measured using Biotech Analyzer AS210 (Sakura Seiki). The results of the fermentation are shown in Table 2.

The composition of the fermentation medium (g/l) is as follows:

| | |
|---|---|
| Glucose | 40 |
| (NH₄)₂SO₄ | 24 |
| K₂HPO₄ | 1.0 |
| MgSO₄ x 7H₂O | 1.0 |
| FeSO₄ x 7H₂O | 0.01 |
| MnSO₄ x 5H₂O | 0.01 |
| Yeast extract | 2.0 |

pH was adjusted to 7.0 by KOH and the medium was autoclaved at 115°C for 10 min. Glucose and MgSO₄ x 7H₂O were sterilized separately. 30 g/l of CaCO₃, which had been dry-heat sterilized at 180°C for 2 hours, was added.

**Table 2**

| Strain | OD₆₀₀ | Lys·HCl, g/l |
|---|---|---|
| WC196(pCABD2, pMW219) | 12.4 | 9.3 |
| WC196(pCABD2, pM-nagE) | 14.5 | 11.3 |

It can be seen from the Table 2, *E. coli* WC196 (pCABD2, pM-*nagE*) causes a higher amount of L-lysine accumulation as compared with *E. coli* WC196 (pCABD2, pMW219).

### Example 6. Effect of increasing the nagE gene expression on L-histidine production.

To test the effect of enhanced expression of the *nagE* gene under the control of a P_{tac} promoter on hidtidine production, the DNA fragments from the chromosome of the above-described *E. coli* strain MG1655Δtdh, rhtA*, P_{tac}nagE can be transferred to histidine-producing *E. coli* strain 80 by P1 transduction (Miller, J.H. (1972) Experiments in Molecular Genetics, Cold Spring Harbor Lab. Press, Plainview, NY). The strain 80 has been described in Russian patent 2119536 and deposited in the Russian National Collection of Industrial Microorganisms (Russia, 117545 Moscow, 1 Dorozhny proezd, 1) on October 15, 1999 under the accession number VKPM B-7270 and then converted to a deposit under the Budapest Treaty on July 12, 2004.

The resulting strain 80 P_{tac}nagE and parent strain 80 can each be cultivated in L broth for 6 hours at 29 °C. Then 0.1 ml of obtained culture can be inoculated into 2 ml of fermentation medium in 20x200mm test tube and cultivated for 65 hours at 29 °C with a rotary shaker (350 rpm). After cultivation, the amount of histidine which has accumulated in the medium can be determined by paper chromatography. The paper can be developed with a mobile phase: n-butanol : acetic acid : water = 4 : 1 : 1 (v/v). A solution of ninhydrin (0.5%) in acetone can be used as a visualizing reagent.

The composition of the fermentation medium (pH 6.0) (g/l):

| | |
|---|---|
| Glucose | 100.0 |
| Mameno (soybean hydrolysate) | 0.2 as total nitrogen |
| L-proline | 1.0 |
| (NH₄)₂SO₄ | 25.0 |
| KH₂PO₄ | 2.0 |
| MgSO₄·•7H₂0 | 1.0 |
| FeSO₄•7H₂·0 | 0.01 |
| MnSO₄ | 0.01 |
| Thiamine | 0.001 |
| Betaine | 2.0 |
| CaCO₃ | 60.0 |

Glucose, proline, betaine and CaCO₃ are sterilized separately. pH is adjusted to 6.0 before sterilization.

### Example 7. Effect of increasing the nagE gene expression on L-phenylalanine production.

To test the effect of enhanced expression of the *nagE* gene under the control of a P*_{tac}* promoter on phenylalanine production, the DNA fragments from the chromosome of the above-described *E. coli* strain MG1655Δtdh, rhtA*, P_{tac}nagE can be transferred to phenylalanine-producing *E. coli* strain AJ12739 by P1 transduction (Miller, J.H. (1972) Experiments in Molecular Genetics, Cold Spring Harbor Lab. Press, Plainview, NY). The strain AJ12739 has been deposited in the Russian National Collection of Industrial Microorganisms (VKPM) (Russia, 113545 Moscow, 1 Dorozhny proezd, 1) on November 6, 2001 under the accession number VKPM B-8197 and then converted to a deposit under the Budapest Treaty on August 23, 2002.

The resulting strain AJ12739 P_{tac}nagE and parent strain AJ12739 can each be cultivated at 37°C for 18 hours in a nutrient broth, and 0.3 ml of the obtained culture can be inoculated into 3 ml of a fermentation medium in a 20 x 200 mm test tube and cultivated at 37°C for 48 hours with a rotary shaker. After cultivation, the amount of phenylalanine which has accumulated in the medium can be determined by TLC. 10 x 15 cm TLC plates coated with 0.11 mm layers of Sorbfil silica gel without fluorescent indicator (Stock Company Sorbpolymer, Krasnodar, Russia) can be used. Sorbfil plates can be developed with a mobile phase: propan-2-ol : ethylacetate : 25% aqueous ammonia : water = 40 : 40 : 7 : 16 (v/v). A solution (2%) of ninhydrin in acetone can be used as a visualizing reagent.

The composition of the fermentation medium (g/l):

| | |
|---|---|
| Glucose | 40.0 |
| (NH₄)₂SO₄ | 16.0 |
| K₂HPO₄ | 0.1 |
| MgSO₄·7H₂O | 1.0 |
| FeSO₄·7H₂O | 0.01 |
| MnSO₄·5H₂O | 0.01 |
| Thiamine HCl | 0.0002 |
| Yeast extract | 2.0 |
| Tyrosine | 0.125 |
| CaCO₃ | 20.0 |

Glucose and magnesium sulfate are sterilized separately. CaCO₃ dry-heat sterilized at 180° for 2 hours. pH is adjusted to 7.0.

### Example 8. Substitution of the native promoter region of the nagE gene in E. coli by hybrid P_{L-tac} promoter.

To substitute the native promoter region of the *nagE* gene, a DNA fragment carrying a hybrid P_{L-tac} promoter and chloramphenicol resistance marker (Cm^{R}) encoded by the *cat* gene was integrated into the chromosome of the *E. coli* MG1655 (ATCC 700926) in place of the native promoter region by the method described by Datsenko K.A. and Wanner B.L. (Proc.Natl.Acad.Sci.USA, 2000, 97, 6640-6645) which is also called as a "Red-mediated integration" or "Red-driven integration". The recombinant plasmid pKD46 (Datsenko, K.A., Wanner, B.L., Proc.Natl.Acad.Sci.USA, 2000, 97, 6640-6645) having a thermosensitive replicon was used as the donor of the phage λ-derived genes responsible for the Red-mediated recombination system. *E. coli* BW25113 containing the recombinant plasmid pKD46 can be obtained from the *E. coli* Genetic Stock Center, Yale University, New Haven, USA, the accession number of which is CGSC7630.

The hybrid P_{L-tac} promoter was synthesized chemically. The nucleotide sequence of the substituted promoter is presented in the Sequence listing (SEQ ID NO: 19). The synthesized DNA fragment containing the hybrid P_{L-tac} promoter contains a *Bgl*II recognition site at the 5'-end thereof, which is necessary for further joining to the *cat* gene and 36 nucleotides homologous to the 5'-terminus of the *nagE* gene introduced for further integration into the bacterial chromosome.

A DNA fragment containing a Cm^{R} marker encoded by the *cat* gene was obtained by PCR using the commercially available plasmid pACYC184 (GenBank/EMBL accession number X06403, "Fermentas", Lithuania) as the template, and primers P17 (SEQ ID NO: 20) and P18 (SEQ ID NO: 21). Primer P17 contains a *Bgl*II recognition site at the 5'-end thereof, which is necessary for further joining to the hybrid P_{L-tac} promoter, and primer P18 contains 36 nucleotides homologous to the region located 120 bp upstream of the start codon of the *nagE* gene, which were introduced into the primer for further integration into the bacterial chromosome.

PCR was provided using the "TermoHybaid PCR Express" amplificator. The reaction mixture (total volume - 50 µl) consisted of 5 µl of 10x PCR-buffer with 15 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added into the reaction mixture as a template DNA for the PCR amplification. The temperature profile was the following: initial DNA denaturation at 95 °C for 5 min, followed by 25 cycles of denaturation at 95 °C for 30 sec, annealing at 55 °C for 30 sec, elongation at 72 °C for 30 sec; and the final elongation at 72 °C for 7 min. Then, the amplified DNA fragment was purified by agarose gel-electrophoresis, extracted using "GenElute Spin Columns" ("Sigma", USA), and precipitated by ethanol.

Each of the two above-described DNA fragments was treated with *Bgl*II restrictase and ligated. The ligation product was amplified by PCR using primers P18 (SEQ ID NO: 21) and P19 (SEQ ID NO: 22).

The amplified DNA fragment was purified by agarose gel-electrophoresis, extracted using "GenElute Spin Columns" ("Sigma", USA), and precipitated by ethanol. The obtained DNA fragment was used for electroporation and Red-mediated integration into the chromosome of *E. coli* MG1655/pKD46.

MG1655/pKD46 cells were grown overnight at 30 °C in the liquid LB-medium containing ampicillin (100 µg/ml), then diluted 1:100 with the SOB-medium (Yeast extract, 5 g/l; NaCl, 0.5 g/l; Tryptone, 20 g/l; KCl, 2.5 mM; MgCl₂, 10 mM) containing ampicillin (100 µg/ml) and L-arabinose (10 mM) (arabinose is used for inducing the plasmid encoding genes of the Red system) and grown at 30 °C to reach the optical density of the culture OD₆₀₀=0.4-0.7. Grown cells from 10 ml of the culture were washed 3 times with the ice-cold de-ionized water, followed by suspending in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions. Shocked cells were added to 1 ml of SOC medium (Sambrook et al, "Molecular Cloning A Laboratory Manual, Second Edition", Cold Spring Harbor Laboratory Press (1989)), incubated 2 hours at 37 °C, and then were spread onto L-agar containing 25 µg/ml of chloramphenicol. Colonies grown within 24 hours were tested for the presence of Cm^{R} marker, instead of the native promoter region of the *nagE* gene by PCR using primers P20 (SEQ ID NO: 23) and P21 (SEQ ID NO: 24). For this purpose, a freshly isolated colony was suspended in 20µl water and then 1 µl of the obtained suspension was used for PCR. The following temperature profile was used: initial DNA denaturation at 95 °C for 10 min; then 30 cycles of denaturation at 95 °C for 30 sec, annealing at 55 °C for 30 sec and elongation at 72 °C for 1 min; the final elongation at 72 °C for 7 min. A few Cm^{R} colonies tested contained the desired ∼1300 bp DNA fragment, confirming the presence of Cm^{R} marker DNA instead of 620 bp native promoter region of *nagE* gene. One of these strains was cured from the thermosensitive plasmid pKD46 by culturing at 37°C and the resulting strain was named as *E. coli* MG1655 P_{L-tac}nagE.

### Example 9. Effect of increasing the nagE gene expression on L-arginine production.

To test the effect of enhanced expression of the *nagE* gene under the control of a P_{L-tac} promoter on arginine production, the DNA fragments from the chromosome of the above-described *E. coli* strain MG1655 P_{L-tac}nagE was transferred to the arginine-producing *E. coli* strain 237 by P1 transduction (Miller, J.H. (1972), Experiments in Molecular Genetics, Cold Spring Harbor Lab. Press, Plainview, NY). The strain 237 has been deposited in the Russian National Collection of Industrial Microorganisms (VKPM) (Russia, 113545 Moscow, 1 Dorozhny proezd, 1) on April 10, 2000 under accession number VKPM B-7925 and then converted to a deposit under the Budapest Treaty on May 18, 2001.

The resulting strain 237 P_{L-tac}nagE and the parent strain 237 were each cultivated at 37 °C for 18 hours in 2 ml of LB nutrient broth, and 0.3 ml of the obtained culture was inoculated into 2 ml of fermentation medium in a 20 x 200 mm test tube, and cultivated at 34 °C for 72 hours on a rotary shaker.

After cultivation, the amount of L-arginine which has accumulated in the medium was determined by paper chromatography using the following mobile phase: butanol : acetic acid : water = 4 : 1 : 1 (v/v). A solution (2%) of ninhydrin in acetone was used as a visualizing reagent. A spot containing L-arginine can be cut off, L-arginine was eluted in 0.5 % water solution of CdCl₂, and the amount of L-arginine was estimated spectrophotometrically at 540 nm. The results of the fermentation are shown in Table 3.

The composition of the fermentation medium (g/l):

| | |
|---|---|
| Glucose | 50.0 |
| (NH4)₂SO₄ | 25.0 |
| KH₂PO₄ | 2.0 |
| MgSO₄·7H₂O | 1.0 |
| Thiamine HCl | 0.0001 |
| Yeast extract | 5.0 |
| L-isoleucine | 0.05 |
| CaCO₃ | 15.0 |

Glucose and magnesium sulfate were sterilized separately. CaCO₃ was dry-heat sterilized at 180 °C for 2 hours. pH was adjusted to 7.0.

**Table 3**

| Strain | OD₅₄₀ | Arg, g/l |
|---|---|---|
| 237 | 20.4±0.4 | 3.5±0.2 |
| 237::P_{L-tac}nagE | 20.2±0.5 | 5.2±0.3 |

It can be seen from the Table 3, *E. coli* 237 P_{L-tac}nagE accumulated a higher amount of L-arginine as compared with *E. coli* 237.

### Example 10. Effect of increasing the nagE gene expression on L-tryptophan production.

To test the effect of enhanced expression of the *nagE* gene under the control of a P_{L-tac} promoter on tryptophan production, the DNA fragments from the chromosome of the above-described *E. coli* strain MG1655P_{L-tac}nagE was transferred to tryptophan-producing *E. coli* SV164 (pGH5) by P1 transduction (Miller, J.H. (1972) Experiments in Molecular Genetics, Cold Spring Harbor Lab. Press, Plainview, NY). The strain SV164 (pGH5) is described in detail in U.S. Patent No. 6,180,373.

The resulting strain SV164(pGH5) P_{L-tac}nagE and the parent strain SV164(pGH5) were each cultivated with shaking at 37 °C for 18 hours in a 3 ml of nutrient broth supplemented with 20 mg/ml of tetracycline (marker of pGH5 plasmid). 0.3 ml of the obtained cultures was inoculated into 3 ml of a fermentation medium containing tetracycline (20 mg/ml) in 20 x 200 mm test tubes, and cultivated at 37 °C for 48 hours with a rotary shaker at 250 rpm. After cultivation, the amount of tryptophan which has accumulated in the medium was determined by TLC as described in Example 7. The composition of the fermentation medium is presented in Table 4. The results of the fermentation are shown in Table 5.

**Table 4**

| Groups | Component | Final concentration, g/l |
|---|---|---|
| A | KH₂PO₄ | 1.5 |
| | NaCl | 0.5 |
| | (NH₄)₂SO₄ | 1.5 |
| | L-Methionine | 0.05 |
| | L-Phenylalanine | 0.1 |
| | L-Tyrosine | 0.1 |
| | Mameno (total N) | 0,07 |
| B | Glucose | 40.0 |
| | MgSO₄·7H₂O | 0.3 |
| C | CaCl₂ | 0.011 |
| D | FeSO₄·7H₂O | 0.075 |
| | Sodium citrate | 1.0 |
| E | Na₂MoO₄·2H₂O | 0.00015 |
| | H₃BO₃ | 0.0025 |
| | CoCl₂·6H₂O | 0.00007 |
| | CuSO₄·5H₂O | 0.00025 |
| | MnCl₂·4H₂O | 0.0016 |
| | ZnSO₄·H₂O | 0.0003 |
| F | Thiamine HCl | 0.005 |
| G | CaCO₃ | 30.0 |
| H | Pyridoxine | 0.03 |

Group A had pH of 7.1, adjusted by NH₄OH. Each group was sterilized separately.

**Table 5**

| Strain | OD₅₄₀ | Trp, g/l |
|---|---|---|
| SV164 (pGH5) | 13.3±0.7 | 4.6±0.1 |
| SV164::P_{L-tac}nagE (pGH5) | 16.2±0.4 | 5.2±0.1 |

It can be seen from the Table 5, *E. coli* SV164 P_{L-tac}nagE (pGH5) accumulated a higher amount of L-tryptophan as compared with *E. coli* SV164 (pGH5).

### Example 11. Effect of increasing the nagE gene expression on L-glutamic acid production.

To test the effect of enhanced expression of the *nagE* gene under the control of a P*_{tac}* promoter on glutamic acid production, the DNA fragments from the chromosome of the above-described *E. coli* strain MG1655Δtdh, rhtA*, P_{tac}nagE can be transferred to glutamic acid-producing *E. coli* strain VL334thrC⁺ by P1 transduction (Miller, J.H. (1972) Experiments in Molecular Genetics, Cold Spring Harbor Lab. Press, Plainview, NY). The strain VL334thrC⁺ is described in detail in EP1172433 and has been deposited in the Russian National Collection of Industrial Microorganisms (Russia, 117545 Moscow, 1 Dorozhny proezd, 1) on December 6, 2004 under the accession number VKPM B-8961 and then converted to a deposit under the Budapest Treaty on December 8, 2004.

The resulting strain VL334thrC⁺ P_{tac}nagE and parent strain VL334thrC⁺ can each be cultivated with shaking at 37 °C for 18 hours in a 3 ml of nutrient broth. 0.3 ml of the obtained cultures can be inoculated into 3 ml of a fermentation medium in 20 x 200 mm test tubes, and cultivated at 37°C for 48 hours with a rotary shaker at 250 rpm.

The composition of the fermentation medium (pH 7.2) (g/l):

| | |
|---|---|
| Glucose | 60.0 |
| Ammonium sulfate | 25.0 |
| KH₂PO₄ | 2.0 |
| MgSO₄ | 1.0 |
| Thiamine | 0.0001 |
| L-isoleucine | 0.05 |
| CaCO₃ | 25.0 |

Glucose and CaCO₃ were sterilized separately.

### Industrial Applicability

According to the present invention, production of L-amino acids, such as L-threonine, L-lysine, L-histidine, L-phenylalanine, L-arginine, L-tryptophan, and L-glutamic acid of a bacterium of the *Enterobacteriaceae* family can be enhanced.

## Claims

1. A method for producing an L-amino acid which comprises cultivating an L-amino acid-producing bacterium of the Enterobacteriaceae family in a culture medium to cause accumulation of the L-amino acid in the culture medium, and isolating the L-amino acid from the culture medium, wherein said bacterium has been modified to enhance an activity of N-acetylglucosamine permease.

2. The method according to claim 1, wherein the activity of N-acetylglucosamine permease is enhanced by increasing the expression of a gene which encodes N-acetylglucosamine permease.

3. The method according to claim 2, wherein said activity of N- acetylglucosamine permease is enhanced by modifying an expression control sequence of the gene encoding N-acetylglucosamine permease or by increasing the copy number of the gene encoding N-acetylglucosamine permease.

4. The method according to claim 1, wherein said bacterium has been additionally modified to enhance an activity of glucokinase.

5. The method according to claim 1, where said bacterium has been additionally modified to enhance an activity of xylose isomerase.

6. The method according to claim 1, wherein the bacterium is selected from the group consisting of the genera Escherichia, Enterobacter, Erwinia, Klebsiella, Pantoea, Providencia, Salmonella, Serratia, Shigella, and Morganella.

7. The method according to claim 1, wherein said gene encodes an N-acetylglucosamine permease selected from the group consisting of:
(A) a protein which comprises the amino acid sequence of SEQ ID NO: 2, and
(B) a variant protein of the amino acid sequence shown in SEQ ID NO: 2, and which has an activity of N-acetylglucosamine permease.

8. The method according to claim 2, wherein said gene encoding N- acetylglucosamine permease comprises a DNA selected from the group consisting of:
(a) a DNA which comprises a nucleotide sequence of nucleotides 1 to 1947 in SEQ ID NO: 1, and
(b) a DNA which is hybridizable with a nucleotide sequence of nucleotides 1- 1947 in SEQ ID NO: 1, or a probe which can be prepared from said nucleotide sequence under stringent conditions, and encodes a protein having an activity of N-acetylglucosamine permease.

9. The method according to claim 8, wherein said stringent conditions comprise those in which washing is performed at 60°C at a salt concentration of 1 x SSC and 0.1 % SDS, for approximately 15 minutes.

10. The method according to claim 1, wherein said bacterium is an L-threonine producing bacterium.

11. The method according to claim 10, wherein said bacterium has been additionally modified to enhance expression of a gene selected from the group consisting of
- the mutant thrA gene which codes for aspartokinase homoserine dehydrogenase I and is resistant to feedback inhibition by threonine,
- the thrB gene which codes for homoserine kinase,
- the thrC gene which codes for threonine synthase,
- the rhtA gene which codes for a putative transmembrane protein,
- the asd gene which codes for aspartate-[beta]-semialdehyde dehydrogenase,
- the aspC gene which codes for aspartate aminotransferase (aspartate transaminase), and combinations thereof.

12. The method according to claim 11, wherein said bacterium has been modified to increase expression of said mutant thrA gene, said thrB gene, said thrC gene, and said rhtA gene.

13. The method according to claim 11, wherein said bacterium has been additionally modified so that the crr gene which codes for catabolite repression regulator is inactivated.

14. The method according to claim 13, wherein said bacterium has been modified to increase expression of said mutant thrA gene, said thrB gene, said thrC gene, and said rhtA gene.

15. The method according to claim 1, wherein said bacterium is an L-lysine producing bacterium.

16. The method according to claim 1, wherein said bacterium is an L-histidine producing bacterium.

17. The method according to claim 1, wherein said bacterium is an L- phenylalanine producing bacterium.

18. The method according to claim 1, wherein said bacterium is an L-arginine producing bacterium.

19. The method according to claim 1, wherein said bacterium is an L-tryptophan producing bacterium.

20. The method according to claim 1, wherein said bacterium is an L-glutamic acid producing bacterium.

21. The method according to claim 1, wherein said L-amino acid is selected from the group consisting of L-threonine, L-lysine, L-histidine, L-phenylalanine, L-arginine, L-tryptophan and L-glutamic acid.

## Patentansprüche

1. Verfahren zur Herstellung einer L-Aminosäure, welches das Kultivieren eines L-Aminosäure herstellenden Bakteriums der Enterobacteriaceae-Familie in einem Kulturmedium, um die Anreicherung der L-Aminosäure in dem Kulturmedium zu verursachen, und das Isolieren der L-Aminosäure aus dem Kulturmedium umfaßt, wobei das Bakterium modifiziert worden ist, um die Aktivität der N-Acetylglucosaminpermease zu verbessern.

2. Verfahren gemäß Anspruch 1, wobei die Aktivität der N-Acetylglucosaminpermease durch die Erhöhung der Expression eines Gens verbessert wird, welches N-Acetylglucosaminpermease codiert.

3. Verfahren gemäß Anspruch 2, wobei die Aktivität der N-Acetylglucosaminpermease durch die Modifizierung einer Expressionskontrollsequenz des Gens verbessert wird, welches die N-Acetylglucosaminpermease codiert, oder durch die Erhöhung der Kopienanzahl des Gens, welches N-Acetylglucosaminpermease codiert.

4. Verfahren gemäß Anspruch 1, wobei das Bakterium zusätzlich modifiziert worden ist, um die Aktivität von Glucokinase zu verbessern.

5. Verfahren gemäß Anspruch 1, wobei das Bakterium zusätzlich modifiziert worden ist, um die Aktivität von Xyloseisomerase zu verbessern.

6. Verfahren gemäß Anspruch 1, wobei das Bakterium ausgewählt ist aus der Gruppe bestehend aus den Genera Escherichia, Enterobacter, Erwinia, Klebsiella, Pantoea, Providencia, Salmonella, Serratia, Shigella und Morganella.

7. Verfahren gemäß Anspruch 1, wobei das Gen eine N-Acetylglucosaminpermease codiert, die aus der Gruppe ausgewählt ist, die besteht aus:
(A) einem Protein, welches die Aminosäuresequenz in SEQ ID Nr. 2 umfaßt, und
(B) einer Proteinvariante der Aminosäuresequenz, die in SEQ ID Nr. 2 gezeigt ist, welche die Aktivität der N-Acetylglucosaminpermease hat.

8. Verfahren gemäß Anspruch 2, wobei das Gen, welches die N-Acetylglucosaminpermease codiert, eine DNA umfaßt, die aus der Gruppe ausgewählt ist, die besteht aus:
(a) einer DNA, welche die Nukleotidsequenz der Nukleotide 1 bis 1947 in SEQ ID Nr. 1 umfaßt, und
(b) einer DNA, die mit einer Nukleotidsequenz der Nukleotide 1 bis 1947 in SEQ ID Nr. 1 hybridisiert, oder mit einer Sonde, die ausgehend von dieser Nukleotidsequenz unter stringenten Bedingungen hergestellt worden ist und die ein Protein codiert, das die Aktivität von N-Acetylglucosaminpermease hat.

9. Verfahren gemäß Anspruch 8, wobei die stringenten Bedingungen solche umfassen, in denen das Waschen bei 60°C bei einer Salzkonzentration von 1 x SSC und 0,1 % SDS für ungefähr 15 Minuten durchgeführt wird.

10. Verfahren gemäß Anspruch 1, wobei das Bakterium ein L-Threonin herstellendes Bakterium ist.

11. Verfahren gemäß Anspruch 10, wobei das Bakterium zusätzlich modifiziert worden ist, um die Expression eines Gens zu verbessern, das aus der Gruppe ausgewählt ist, die besteht aus:
- dem mutierten thrA-Gen, welches für Aspartokinasehomoserindehydrogenase I codiert und gegenüber einer Rückkopplungsinhibierung durch Threonin resistent ist,
- dem thrB-Gen, welches für Homoserinkinase codiert,
- dem thrC-Gen, welches für Threoninsynthase codiert,
- dem rhtA-Gen, welches für ein mutmaßliches Transmembranprotein codiert,
- dem asd-Gen, welches für eine Aspartat-[beta]-semialdehyddehydrogenase codiert,
- dem aspC-Gen, welches für eine Aspartataminotransferase (Aspartattransaminase) codiert sowie Kombinationen daraus.

12. Verfahren gemäß Anspruch 11, wobei das Bakterium modifiziert worden ist, um die Expression des mutierten thrA-Gens, des thrB-Gens, des thrC-Gens und des rhtA-Gens zu erhöhen.

13. Verfahren gemäß Anspruch 11, wobei das Bakterium zusätzlich modifiziert worden ist, so daß das crr-Gen, welches für einen Katabolitrepressionsregulator codiert, inaktiviert ist.

14. Verfahren gemäß Anspruch 13, wobei das Bakterium modifiziert worden ist, um die Expression des mutierten thrA-Gens, des thrB-Gens, des thrC-Gens und des rhtA-Gens zu erhöhen.

15. Verfahren gemäß Anspruch 1, wobei das Bakterium ein L-Lysin herstellendes Bakterium ist.

16. Verfahren gemäß Anspruch 1, wobei das Bakterium ein L-Histidin herstellendes Bakterium ist.

17. Verfahren gemäß Anspruch 1, wobei das Bakterium ein L-Phenylalanin herstellendes Bakterium ist.

18. Verfahren gemäß Anspruch 1, wobei das Bakterium ein L-Arginin herstellendes Bakterium ist.

19. Verfahren gemäß Anspruch 1, wobei das Bakterium ein L-Tryptophan herstellendes Bakterium ist.

20. Verfahren gemäß Anspruch 1, wobei das Bakterium ein L-Glutaminsäure herstellendes Bakterium ist.

21. Verfahren gemäß Anspruch 1, wobei die L-Aminosäure ausgewählt ist aus der Gruppe bestehend aus L-Threonin, L-Lysin, L-Histidin, L-Phenylalanin, L-Arginin, L-Tryptophan und L-Glutaminsäure.

## Revendications

1. Procédé destiné à produire un acide aminé L qui comprend la culture d'une bactérie produisant un acide aminé L de la famille des Enterobacteriaceae dans un milieu de culture afin de provoquer une accumulation de l'acide aminé L dans le milieu de culture, et l'isolement de l'acide aminé L à partir du milieu de culture, dans lequel ladite bactérie a été modifiée afin de renforcer une activité de N-acétylglucosamine perméase.

2. Procédé selon la revendication 1, dans lequel l'activité de N-acétylglucosamine perméase est renforcée par augmentation de l'expression d'un gène qui code pour une N-acétylglucosamine perméase.

3. Procédé selon la revendication 2, dans lequel ladite activité de N-acétylglucosamine perméase est renforcée par modification d'une séquence de contrôle de l'expression du gène codant pour une N-acétylglucosamine perméase ou par augmentation du nombre de copies du gène codant pour une N-acétylglucosamine perméase.

4. Procédé selon la revendication 1, dans lequel ladite bactérie a de plus été modifiée afin de renforcer une activité de glucokinase.

5. Procédé selon la revendication 1, dans lequel ladite bactérie a de plus été modifiée afin de renforcer une activité de xylose isomérase.

6. Procédé selon la revendication 1, dans lequel la bactérie est choisie dans le groupe constitué du genre Escherichia, Enterobacter, Erwinia, Klebsiella, Pantoea, Providencia, Salmonella, Serratia, Shigella et Morganella.

7. Procédé selon la revendication 1, dans lequel ledit gène code pour une N-acétylglucosamine perméase choisie dans le groupe constitué de :
(A) une protéine qui comprend la séquence d'acides aminés de SEQ ID N° :2, et
(B) une protéine variante de la séquence d'acides aminés montrée dans SEQ ID N° :2, et qui possède une activité de N-acétylglucosamine perméase.

8. Procédé selon la revendication 2, dans lequel ledit gène codant pour une N-acétylglucosamine perméase comprend un ADN choisi dans le groupe constitué de :
(a) un ADN qui comprend une séquence nucléotidique des nucléotides 1 à 1947 dans SEQ ID N° :1, et
(b) un ADN qui peut s'hybrider avec une séquence nucléotidique des nucléotides 1 à 1947 dans SEQ ID N° :1, ou une sonde qui peut être préparée à partir de ladite séquence nucléotidique dans des conditions stringentes, et qui code pour une protéine possédant une activité de N-acétylglucosamine perméase.

9. Procédé selon la revendication 8, dans lequel lesdites conditions stringentes comprennent celles dans lesquelles le lavage est réalisé à 60°C à une concentration saline de 1 x SSC et 0,1 % SDS, pendant environ 15 minutes.

10. Procédé selon la revendication 1, dans lequel ladite bactérie est une bactérie produisant de la L-thréonine.

11. Procédé selon la revendication 10, dans lequel ladite bactérie a de plus été modifiée afin de renforcer l'expression d'un gène choisi dans le groupe constitué
- du gène thrA mutant qui code pour une aspartokinase homosérine déshydrogénase I et est résistant à l'inhibition par rétrocontrôle par la thréonine,
- du gène thrB qui code pour une homosérine kinase,
- du gène thrC qui code pour une thréonine synthase,
- du gène rhtA qui code pour une protéine transmembranaire putative,
- du gène asd qui code pour une aspartate- [béta] - semialdéhyde déshydrogénase,
- du gène aspC qui code pour une aspartate aminotransférase (aspartate transaminase), et des combinaisons de celles-ci.

12. Procédé selon la revendication 11, dans lequel ladite bactérie a été modifiée afin d'augmenter l'expression dudit gène thrA mutant, dudit gène thrB, dudit gène thrC, et dudit gène rhtA.

13. Procédé selon la revendication 11, dans lequel ladite bactérie a de plus été modifiée afin que le gène crr qui code pour un régulateur de répression du catabolite soit inactivé.

14. Procédé selon la revendication 13, dans lequel ladite bactérie a été modifiée afin d'augmenter l'expression dudit gène thrA mutant, dudit gène thrB, dudit gène thrC, et dudit gène rhtA.

15. Procédé selon la revendication 1, dans lequel ladite bactérie est une bactérie produisant de la L-lysine.

16. Procédé selon la revendication 1, dans lequel ladite bactérie est une bactérie produisant de la L-histidine.

17. Procédé selon la revendication 1, dans lequel ladite bactérie est une bactérie produisant de la L-phénylalanine.

18. Procédé selon la revendication 1, dans lequel ladite bactérie est une bactérie produisant de la L-arginine.

19. Procédé selon la revendication 1, dans lequel ladite bactérie est une bactérie produisant du L-tryptophane.

20. Procédé selon la revendication 1, dans lequel ladite bactérie est une bactérie produisant de l'acide L glutamique.

21. Procédé selon la revendication 1, dans lequel ledit acide aminé L est choisi dans le groupe constitué de la L-thréonine, la L-lysine, la L-histidine, la L-phénylalanine, la L-arginine, le L-tryptophane et de l'acide L glutamique.
